(19) Europäisches Patentamt
European Patent Office
Office européen des brevets

(11) **EP 4 424 703 A1**

(12) **EUROPEAN PATENT APPLICATION**

(43) Date of publication:
04.09.2024 Bulletin 2024/36

(21) Application number: 23159544.8

(22) Date of filing: 02.03.2023

(51) International Patent Classification (IPC):
*C07K 14/31* (2006.01)   *C07K 14/705* (2006.01)
*A61P 25/00* (2006.01)   *A61P 25/16* (2006.01)
*A61P 35/00* (2006.01)   *A61K 47/64* (2017.01)

(52) Cooperative Patent Classification (CPC):
**A61P 25/00; C07K 14/31; C07K 16/2881;**
C07K 14/70582; C07K 2317/33; C07K 2317/77;
C07K 2317/92; C07K 2318/20; C07K 2319/00;
C07K 2319/21; C07K 2319/31

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Amylonix AB**
**121 36 Johanneshov (SE)**

(72) Inventors:
• **STÅHL, Stefan**
**111 40 Stockholm (SE)**
• **LÖFBLOM, John**
**146 54 Tullinge (SE)**

• **LINDBERG, Hanna**
**116 22 Stockholm (SE)**
• **HJELM, Linnea**
**170 70 Solna (SE)**
• **FLEMMING SVEDMARK, Siri**
**112 45 Stockholm (SE)**

(74) Representative: **Kransell & Wennborg KB**
**P.O. Box 27834**
**115 93 Stockholm (SE)**

Remarks:
The complete document including Reference
Table(s) and the Sequence Listing(s) can be
downloaded from the EPO website

(54) **POLYPEPTIDES FOR TRANSFERRIN RECEPTOR-MEDIATED TRANSCYTOSIS**

(57) The present disclosure provides a transferrin receptor 1-binding polypeptide.

Fig. 1

**Description**

**TECHNICAL FIELD**

The present disclosure relates to the delivery of drugs to the brain.

**BACKGROUND**

Antibodies and other biological drugs have had a profound impact on the treatment of many different diseases, such as different forms of cancers and autoimmune disorders. However, for diseases of the central nervous system (CNS), the restricted uptake over the blood-brain barrier (BBB) makes development of therapeutics and diagnostic tools more challenging.

The BBB protects the sensitive neuronal environment by substantially decreasing paracellular diffusion of substances from blood, and various receptors and transporters control the transcytosis of molecules in and out between the brain and blood compartments [1-3]. Transport can either be by passive or active transportation, including receptor-mediated transcytosis (RMT) [2]. It has been shown that about 0.1-0.2% of systemically administrated antibodies pass into the CSF [4], which is potentially too low to achieve efficient disease-modifying effects [5]. Therefore, efforts have been made towards finding a transportation system across the BBB for biological drugs. One of the more well-studied approaches is RMT via the transferrin receptor-1 (TfR) that naturally transports transferrin (Tf) across the BBB to the neuronal environment. An example is the anti-murine TfR-specific 8D3 shuttle antibody [6], which has shown up to 8o-fold increased uptake to the brain in rodent models [7]. For antibodies targeting TfR, several parameters have to be considered, such as affinity [8], valency [7,9], and antibody effector functions for minimizing potential adverse effects [10].

In addition to antibodies and antibody derivatives [10-12], shuttles based on modified fragment crystallisable (Fc) from antibodies [13] and alternative scaffolds are emerging. The alternative scaffolds are including a variety of protein structures, such as e.g. VNARs [14] and cysteine-dense peptides [15]. With a monovalent format and lack of Fc they might potentially circumvent some limitations and safety liabilities previously seen for bivalent antibodies [16,17]. Moreover, the small size of alternative scaffolds might benefit biodistribution and targeting due to more rapid diffusion in the brain parenchyma as has been shown for single-chain variable fragment (scFv) when compared to full-length antibodies [18].

Affibody molecules are small (6 kDa) affinity proteins, which fold into a three-helical structure and typically demonstrate high thermal stability, high solubility and efficient and complete refolding after denaturation [19]. Production is efficient in prokaryotic *Escherichia coli* and the small size allows for production by solid-phase chemical peptide synthesis, enabling straight-forward incorporation of unnatural amino acids [19,20]. Affibodies with new specificities are generated by directed evolution (e.g. phage and bacterial display [19,21,22]) and binders to over 60 different targets have been reported in the literature [19]. The most advanced affibody molecule in clinical testing is a binder with femtomolar affinity for interleukin 17-A (IL17-A) [19,23]. Results from phase I and II clinical trials show outstanding efficacy in various IL17-driven autoimmune disorders, and excellent safety profile [23]. Selections from affibody libraries against intrinsically disordered neurodegenerative peptides have resulted in dimeric variants of the affibody scaffold with an unusual three-dimensional structure and mode of binding. The atypical dimeric binders are denoted sequestrins [24] and an example is a sequestrin ($Z_{SYM73}$) targeting amyloid beta with subnanomolar affinity [25]. In (APP)/PS1 transgenic AD animal models, $Z_{SYM73}$ restored the cognitive function, inhibited amyloid beta aggregation, and eliminated neurotoxic effects [25]. Compared to antibodies, the small size of affibodies and sequestrins results in improved tissue penetration [19], but transportation across the BBB is still limited, corresponding to a bioavailability of around 0.1-0.2% in CSF [25]. In a follow-up study, $Z_{SYM73}$ was therefore fused to a scFv(8D3) brain shuttle, resulting in 9-fold increase in CSF bioavailability [26].

**SUMMARY**

Encouraged by the above-mentioned results, the present inventors have selected affibody molecules against TfR using phage display technology. Two of the binders ($Z_{TfR\#14}$ and $Z_{TfR\#18}$) demonstrated cross-reactivity to both human and murine TfR and displayed non-competitive binding with human transferrin. These two binders were further refined by single amino acid mutagenesis and evaluated in terms of different characteristics such as, thermal stability and cell binding. Finally, the top candidates were investigated for their ability of RMT using TfR-expressing brain endothelial cell lines in a recombinant silk-based *in vitro* assay, in which an increased uptake to the basal side for the TfR-specific affibodies was observed.

Another mutagenesis study of $Z_{TfR\#18}$ and $Z_{TfR\#18}$ was then performed to evaluate the binding contribution of amino acids and how substituting to other amino acids affects binding to TfR.

The new TfR-specific affibody molecules identified herein can act as small brain-shuttles for increasing uptake of various compounds to the central nervous system.

Accordingly, the present disclosure provides the following itemized listing of embodiments.

1. A transferrin receptor 1-binding polypeptide comprising the amino acid sequence

$X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}X_{32}X_{33}X_{34}X_{35}$ (SEQ ID NO:1) wherein

$X_9$ is A, Q, S, H or E,
$X_{10}$ is G, D, E, N or Q,
$X_{11}$ is R, Q, G, H or K,
$X_{12}$ is A, V or T,
$X_{13}$ is K, N or R,
$X_{14}$ is F, L, S, H, I or Y,
$X_{15}$ is E, D or V,
$X_{16}$ is I, N or T,
$X_{17}$ is Y, L or F,
$X_{18}$ is Q, N, E, T, K or D,
$X_{19}$ is L, P or Q,
$X_{20}$ is P, S, Q or T,
$X_{21}$ is N, K, SorY,
$X_{22}$ is L, M or Q,
$X_{24}$ is M, N, R, K, Q or H,
$X_{25}$ is F, I, L, Y, R, K, T, S, Q or N,
$X_{26}$ is Q, P or H,
$X_{27}$ is L, H, V, Q, R or K,
$X_{28}$ is F, V, Y or H,
$X_{29}$ is A or T,
$X_{30}$ is F, I or Y,
$X_{31}$ is H, R, Q, Y, L, D or N,
$X_{32}$ is H, Y, P, N, L, R, E or K,
$X_{33}$ is S, G, T or I,
$X_{34}$ is L, S, F or V and
$X_{35}$ is F, L, K, Y, R, S, D, E, N.

2. The polypeptide of item 1, further comprising the amino acid sequence $X_4X_5X_6X_7X_8$ at the N terminus of SEQ ID NO:1, wherein

$X_4$ is K, R or E, preferably K,
$X_5$ is F, I, L or S, preferably F or S, more preferably F,
$X_6$ is N or S, preferably N,
$X_7$ is K or R, preferably K, and
$X_8$ is E, V or D, preferably E or V, more preferably E.

3. The polypeptide of item 2, wherein the N terminus of SEQ ID NO:1 is further extended by the sequence VDN (SEQ ID NO:3).

4. The polypeptide of any one of the preceding items, further comprising the amino acid sequence $X_{36}X_{37}X_{38}X_{39}X_{40}$ at the C terminus of SEQ ID NO:1, wherein

$X_{36}$ is D, E or V, preferably D or V,
$X_{37}$ is D or N, preferably D,
$X_{38}$ is P or S, preferably P,
$X_{39}$ is S, T, I or G, preferably S or T, more preferably S, and
$X_{40}$ is Q, L, E or H, preferably Q, E or H, more preferably Q or H.

5. The polypeptide of item 4, wherein the C terminus of SEQ ID NO:1 is further extended by the sequence $X_{41}X_{42}X_{43}X_{44}X_{45}X_{46}X_{47}X_{48}X_{49}X_{50}X_{51}X_{52}X_{53}X_{54}$, wherein

$X_{41}$ is S, N, R or G, preferably S or N,
$X_{42}$ is A, V or T, preferably A,
$X_{43}$ is N or D, preferably N,
$X_{44}$ is L, M or F, preferably L or M,
$X_{45}$ is L, P or Q, preferably L or P,
$X_{46}$ is A, P, G, T or S, preferably A, P or T,
$X_{47}$ is E, D or K, preferably E or K,
$X_{48}$ is A, V or T, preferably A,
$X_{49}$ is K, R, N or I, preferably K or R,
$X_{50}$ is K, N, E or M, preferably K or M,
$X_{51}$ is L, I, Q or P, preferably L,
$X_{52}$ is N, I, D or K, preferably N,
$X_{53}$ is D, N or E, preferably D or N, and
$X_{54}$ is A, T, P or V, preferably A.

6. The polypeptide of item 5, wherein the C terminus of SEQ ID NO:1 is still further extended by the sequence QAX$_{57}$K (SEQ ID NO:4), wherein $X_{57}$ is P or S, preferably P.

7. The polypeptide of any one of the preceding items, wherein

$X_9$ is A, Q, S, H or E,
$X_{11}$ is R, H or K,
$X_{12}$ is A, V or T,
$X_{13}$ is K or R,
$X_{14}$ is F, H, I or Y,
$X_{15}$ is E or V,
$X_{16}$ is I,
$X_{17}$ is Y, L or F,
$X_{18}$ is Q, N, E, T, K or D,
$X_{19}$ is L or P,
$X_{20}$ is P, S or T,
$X_{21}$ is N,
$X_{22}$ is L, M or Q,
$X_{25}$ is F, I, L, R, K, T, S, Q or N,
$X_{26}$ is Q,
$X_{27}$ is L, H, Q, R or K,
$X_{30}$ is F or Y,
$X_{31}$ is H, Q, D or N,
$X_{33}$ is S or G,
$X_{34}$ is L or S and/or
$X_{35}$ is F, L, K, Y, S, D, E or N.

8. The polypeptide of any one of the preceding items, wherein

$X_9$ is A, Q or H,
$X_{10}$ is G, D or E,
$X_{11}$ is R or H,
$X_{12}$ is A or T,
$X_{13}$ is K or R,
$X_{14}$ is F or H,
$X_{15}$ is E or V,
$X_{16}$ is I,
$X_{17}$ is Y or F,
$X_{18}$ is Q, N or E,
$X_{19}$ is L or P,
$X_{20}$ is P or T,
$X_{21}$ is N,
$X_{24}$ is M,

$X_{25}$ is F, I, R or K, preferably F, R or K
$X_{26}$ is Q,
$X_{27}$ is L, R, H or K,
$X_{28}$ is F,
$X_{30}$ is F or Y,
$X_{31}$ is H, Q or D,
$X_{32}$ is H, R or K,
$X_{33}$ is S,
$X_{34}$ is L or S and/or
$X_{35}$ is F, D or E.

9. A transferrin receptor 1-binding polypeptide comprising the amino acid sequence
$RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}X_{32}X_{33}X_{34}X_{35}$ (SEQ ID NO:2) wherein

$X_{10}$ is H, L, R or Y,
$X_{11}$ is N or I,
$X_{12}$ is A, S, E or V,
$X_{13}$ is F or H,
$X_{14}$ is M or H,
$X_{16}$ is I or T,
$X_{17}$ is H, P, N or R,
$X_{18}$ is F, H, Y or S,
$X_{19}$ is L or Q,
$X_{20}$ is P, S or Q,
$X_{21}$ isN orY,
$X_{22}$ is L, M, I or Q,
$X_{23}$ is N, SorY,
$X_{24}$ is I, F or V,
$X_{25}$ is D or V,
$X_{26}$ is Q, R or H,
$X_{27}$ is N, I or Y,
$X_{28}$ is Y or N,
$X_{29}$ is A or T,
$X_{31}$ is D, E, G or Y,
$X_{32}$ is F, I or L,
$X_{33}$ is S or R,
$X_{34}$ is L or F and
$X_{35}$ is K, M or R.

10. The polypeptide of item 9, further comprising the amino acid sequence $X_4X_5X_6X_7X_8$ at the N terminus of SEQ ID NO:2, wherein

$X_4$ is K or I, preferably K,
$X_5$ is F,
$X_6$ is N,
$X_7$ is K, and
$X_8$ is E.

11. The polypeptide of item 10, wherein the N terminus of SEQ ID NO:2 is further extended by the sequence VDN.

12. The polypeptide of any one of items 9-11, further comprising the amino acid sequence $X_{36}X_{37}X_{38}X_{39}X_{40}$ at the C terminus of SEQ ID NO:2, wherein

$X_{36}$ is D,
$X_{37}$ is D or E, preferably D,
$X_{38}$ is P, L or T, preferably P or T,
$X_{39}$ is S, and
$X_{40}$ is Q, L or H, preferably Q.

13. The polypeptide of item 12, wherein the C terminus of SEQ ID NO:2 is further extended by the sequence $X_{41}X_{42}X_{43}X_{44}X_{45}X_{46}X_{47}X_{48}X_{49}X_{50}X_{51}X_{52}X_{53}X_{54}$, wherein

$X_{41}$ is S,
$X_{42}$ is A,
$X_{43}$ is N or T, preferably N,
$X_{44}$ is L,
$X_{45}$ is L,
$X_{46}$ is A,
$X_{47}$ is E,
$X_{48}$ is A or T,
$X_{49}$ is K,
$X_{50}$ is K, E or M, preferably K or M,
$X_{51}$ is L or P, preferably L,
$X_{52}$ is N,
$X_{53}$ is D or Y, and
$X_{54}$ is A or T, preferably A.

14. The polypeptide of item 13, wherein the C terminus of SEQ ID NO:2 is still further extended by the sequence $QX_{56}X_{57}K$ (SEQ ID NO:5), wherein $X_{56}$ is A or V, preferably A, and $X_{57}$ is P or S, preferably P.

15. The polypeptide of any one of the preceding items, wherein

$X_{10}$ is H, R or Y,
$X_{12}$ is A, SorV,
$X_{13}$ is F,
$X_{16}$ is I,
$X_{17}$ is H or N,
$X_{18}$ is F or H,
$X_{19}$ is L,
$X_{20}$ is P or Q,
$X_{21}$ isN orY,
$X_{22}$ is L, I or Q,
$X_{23}$ is N or S,
$X_{24}$ is I,
$X_{25}$ is D or V,
$X_{26}$ is Q or H,
$X_{27}$ is N or I,
$X_{28}$ is Y or N,
$X_{29}$ is A or T,
$X_{31}$ is D or Y,
$X_{32}$ is F, I or L,
$X_{33}$ is S,
$X_{34}$ is L or F and/or
$X_{35}$ is K or M.

16. A fusion protein or conjugate comprising:

- a first part comprising a polypeptide according to any one of items 1-15; and
- a second part having a therapeutic activity or binding a diagnostic target.

17. The fusion protein of item 16, wherein the therapeutic activity is based on binding to a target selected from the group consisting of amyloid β, Tau, apolipoprotein E (ApoE), beta-secretase 1 (BACE1), gamma-secretase, complement factor C1s, presenilin 1, presenilin 2, nicastrin, alpha-synuclein, sortilin, progranulin, prosaposin and Bri.

18. The polypeptide, fusion protein or conjugate of any one of the preceding items for use as a medicament.

19. A pharmaceutical composition comprising the polypeptide, fusion protein or conjugate of any one of the preceding items.

20. The polypeptide, fusion protein, conjugate or pharmaceutical composition of any one of the preceding items for use in a method of treatment of a disorder, preferably a neurological disorder.

21. The polypeptide, fusion protein, conjugate or pharmaceutical composition for use according to item 20, wherein the disorder is Parkinson's disease or dementia.

22. The polypeptide, fusion protein, conjugate or pharmaceutical composition for use according to item 21, wherein said dementia is Lewy body dementia or Alzheimer's disease.

23. The polypeptide, fusion protein, conjugate or pharmaceutical composition for use according to item 20, wherein the disorder is a brain cancer, such as glioma.

24. The polypeptide, fusion protein, conjugate or pharmaceutical composition for use according to item 20, wherein the disorder is glaucoma.

25. The polypeptide, fusion protein, conjugate or pharmaceutical composition for use according to item 20, wherein the disorder is multiple sclerosis (MS).

**BRIEF DESCRIPTION OF THE FIGURES**

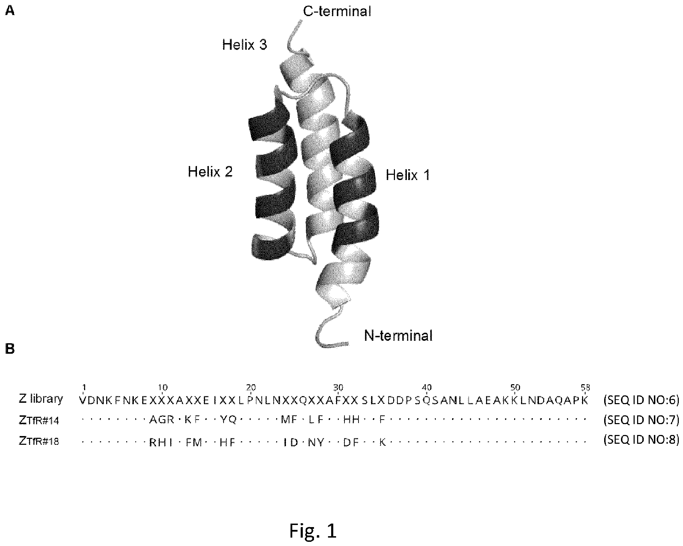

Figure 1. **(A)** Affibody structure with 14 randomised positions (black) in helices 1 and 2 (PDB:2B89). **(B)** Z library sequence (SEQ ID NO:6) with randomised positions marked with "X" aligned to sequences for $Z_{TfR\#14}$ (SEQ ID NO:7) and $Z_{TfR\#18}$ (SEQ ID NO:8).

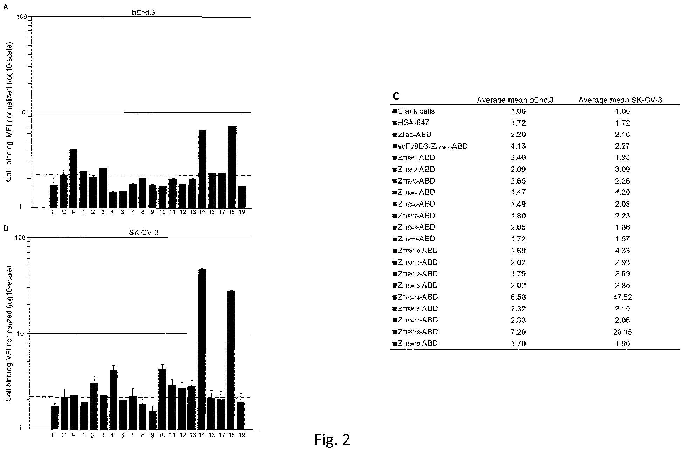

Figure 2A-B. Analysis by flow cytometry of $Z_{TfR}$-ABD constructs at 1 $\mu$M on **(A)** murine hTfR expressing bEnd.3 cells and **(B)** human TfR-expressing SK-OV-3 cells. The bar chart show MFI (mean fluorescent intensity) of TfR-binding and signals are normalized to blank cells for respective cell line. The first bar in both groups is negative control with cells incubated only with secondary reagent (*H). The dashed line represents signal from the negative control affibody molecule (Ztaq-ABD) (*C). A construct scFv8D3-$Z_{SYM73}$-ABD (*P) with a scFv fragment of the murine TfR-specific antibody 8D3 is included as positive control.

Figure 2C. Mean fluorescent intensity (MFI) for cellular binding between the [His6-$Z_{TfR}$-ABD] and TfR-expressing cells bEnd.3 and SK-OV-3. Normalised to the blank cells without any fluorophore added for each cell line. Controls with cells and only HSA-647 fluorophore, the control affibody Ztaq or mTfR specific scFv8D3-$Z_{SYM73}$-ABD are included. Detection of binding was done by HSA-647 measured at 640 nm laser and 660 nm BP filter.

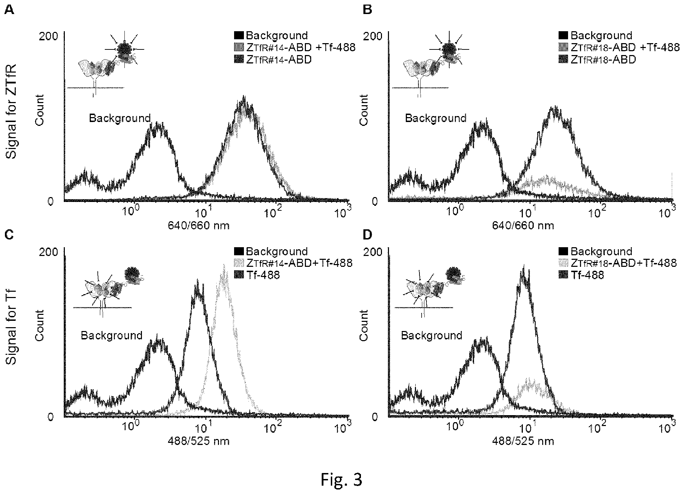

Figure 3. Flow cytometry of SK-OV-3 cells. **(A-B)** Fluorescence from cells corresponding to affibody-binding. $Z_{TfR\#14}$-ABD or $Z_{TfR\#18}$-ABD co-incubated with molar excess of transferrin-AF488 (Tf-488). Cells incubated with only affibody are shown in darker grey. Cells incubated with affibody and Tf-488 are shown in light grey (only at highest molar excess only shown). The $Z_{TfR}$-ABD is detected by HSA-647 at 640/660 nm laser and filter. **(C-D)** Fluorescence from cells corresponding to Tf-488 binding. $Z_{TfR\#14}$-ABD or $Z_{TfR\#18}$-ABD co-incubated with molar excess of Tf-488. Cells incubated with only Tf-488 are shown in dark grey. Cells incubated with affibody and Tf-488 are shown in light grey (highest molar excess only shown). Tf-488 is detected at 488/525 nm laser and filter. All samples are measured for 20,000 cells.

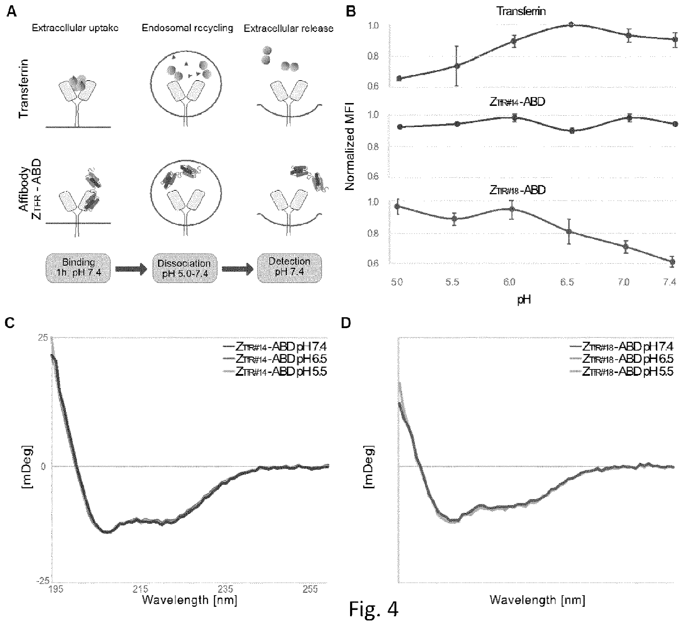

Figure 4. Evaluation for pH-dependent binding of the affibody constructs. **(A)** Schematic illustration over the experimental flow cytometric procedure. The sample is subjected to cells at physiological pH, before washing and divided into different samples for dissociation at different pH for 30 min. The samples are finally washed in pH 7.4 and analysed in the flow cytometer. **(B)** The signal obtained after incubation at different pH for Tf, $Z_{TfR\#14}$, and $Z_{TfR\#18}$, respectively. **(C-D)** Measurement of secondary structure by circular dichroism spectroscopy for $Z_{TfR\#14}$, and $Z_{TfR\#18}$ at pH 5.5, 6.5 and 7.4.

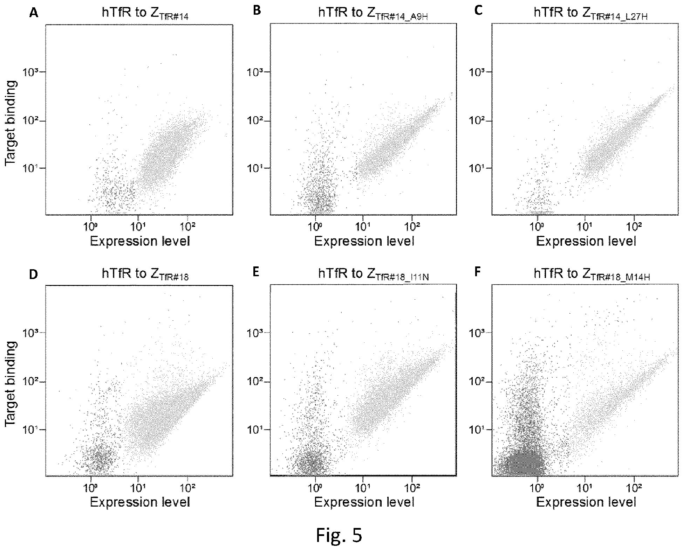

Figure 5. Flow cytometry on *E. coli* displaying original and mutated variants of $Z_{TfR\#14}$, and $Z_{TfR\#18}$ in fusion to an albumin-binding domain (ABD). Human TfR-binding on y-axis and surface expression level measured by binding to fluorescently labelled HSA on x-axis. **(A-C)** *E. coli* displaying $Z_{TfR\#14}$, $Z_{TfR\#14\_A9H}$, and $Z_{TfR\#14\_L27H}$ incubated with 100 nM hTfR. **(D-F)** *E. coli* displaying $Z_{TfR\#18}$, $Z_{TfR\#18\_I11N}$, and $Z_{TfR\#18\_M14H}$ incubated with 75 nM hTfR. Light grey includes HSA-positive ABD-expressing cell population.

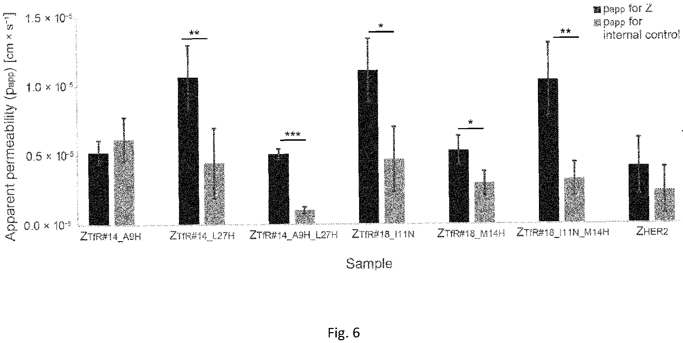

Figure 6. Apparent permeability ($p_{app}$) for transcytosis over a bEnd.3 cell barrier formed on recombinant silk membranes. Each sample is analyzed in at least triplicate and compared with the simultaneously added internal control in a twosided students t-test (*p-value < 0.05, **p-value < 0.01, ***p-value < 0.005). The $Z_{HER2}$ control is targeting the $HER_2$ receptor.

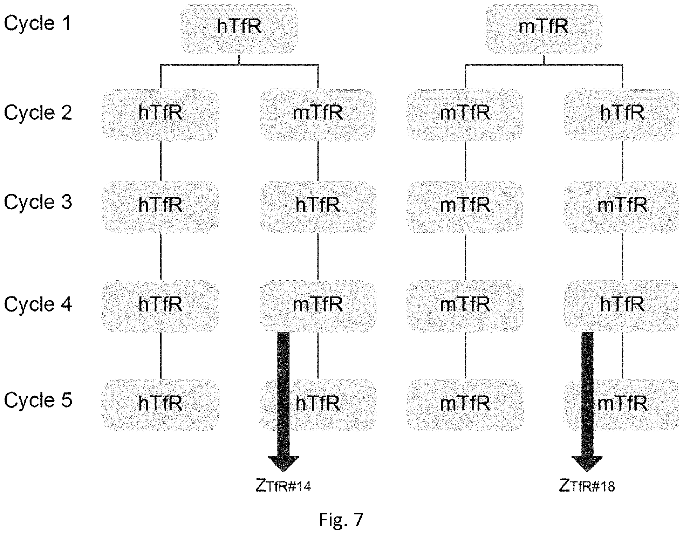

Figure 7. Selection from phage library was performed in four tracks with different strategies. Clones from selection cycle 4 and 5 were analysed and characterised. $Z_{TfR\#14}$ and $Z_{TfR\#18}$ are derived from the fourth round of selections from the tracks with alternating murine and human TfR.

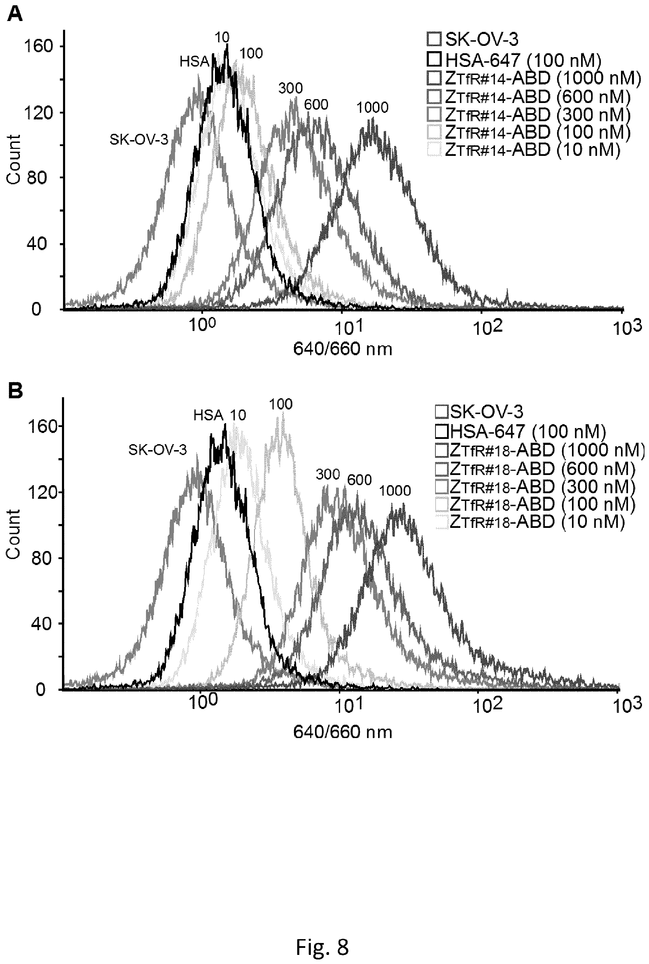

Figure 8. Concentration-dependent binding of the constructs determined by flow cytometry for **(A)** $Z_{TfR\#14}$-ABD and **(B)** $Z_{TfR\#18}$-ABD in the range of 10-1000 nM (light to dark) detected by HSA-647 to the human expressing TfR cell line SK-OV-3. The y-axis shows counted cells and x-axis the fluorescent signal from HSA-647 measured at 640 nm laser and 660 nm BP filter. In total 20,000 cells were analysed per sample.

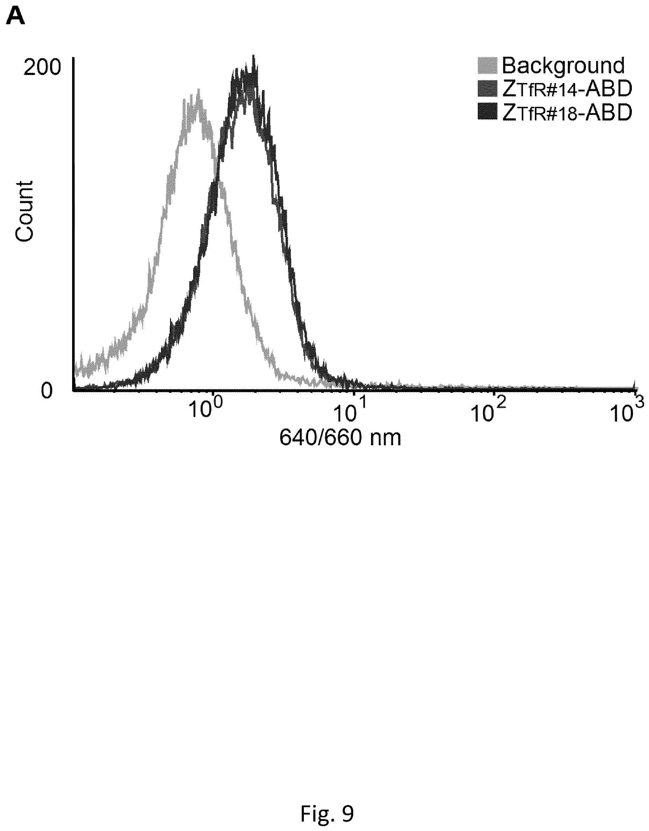

Figure 9. Flow cytometry showing binding to hCMEC/D3 cells (light grey) for 300 nM of $Z_{TfR\#14}$-ABD (dark grey) and $Z_{TfR\#18}$-ABD (dark grey) with detection via HSA-647 at 640 nm laser and 660 nm BP filter. In total 20,000 cells were analysed per sample.

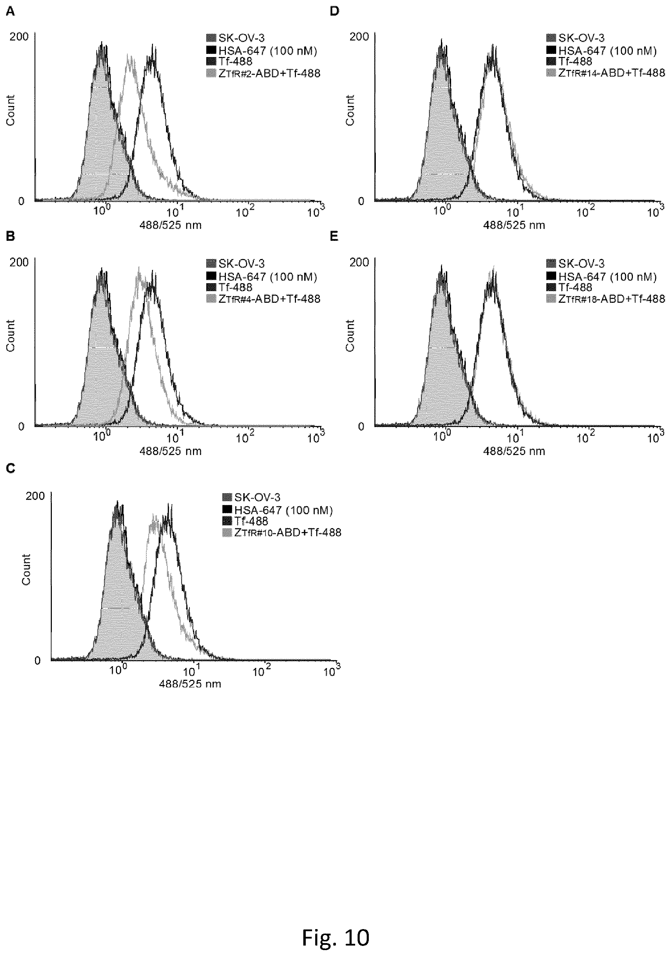

Figure 10. Flow cytometry of SK-OV-3 cells. Fluorescence from cells corresponding to transferrin-AF488 (Tf-488)-binding. $Z_{TfR\#14}$-ABD or $Z_{TfR\#18}$-ABD co-incubated with different molar excess of Tf-488. Only cells are shown in grey and cells incubated with only secondary reagent are shown in black with filled grey curves. Cells incubated with only Tf-488 are shown in dark grey. Cells incubated with affibody and Tf are shown in light grey. Tf-488 is detected at 488/525 nm laser and filter. **(A)** $Z_{TfR\#2}$-ABD **(B)** $Z_{TfR\#4}$-ABD **(C)** $Z_{TfR\#10}$-ABD, **(D)** $Z_{TfR\#14}$-ABD and **(E)** $Z_{TfR\#18}$-ABD co-incubated with Tf-488.

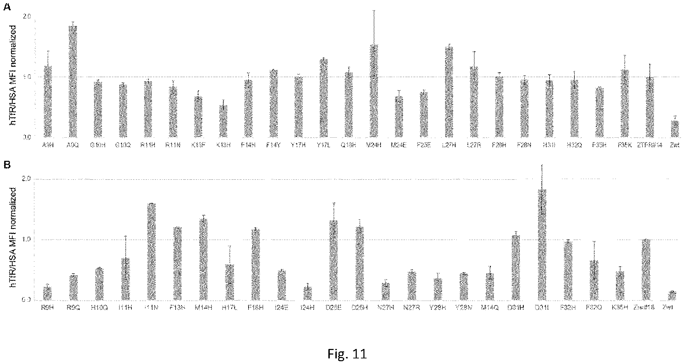

Figure 11. Mean fluorescence intensity (MFI) from flow cytometry on *E. coli* cells displaying single amino acid mutants of $Z_{TfR\#14}$ or $Z_{TfR\#18}$ in fusion to an albumin-binding domain. A negative control Zwt was included for binding of hTfR to *E. coli.* Cells were incubated with labelled human TfR and fluorescently labelled HSA. MFI values are normalized by hTfR binding to expression levels (measured by HSA) and normalized with the signal for the original binder ($Z_{TfR\#14}$ or $Z_{TfR\#18}$). **(A)** Mutants of $Z_{TfR\#14}$ incubated with 100 nM hTfR and **(B)** Mutants of $Z_{TfR\#18}$ incubated with 75 nM hTfR.

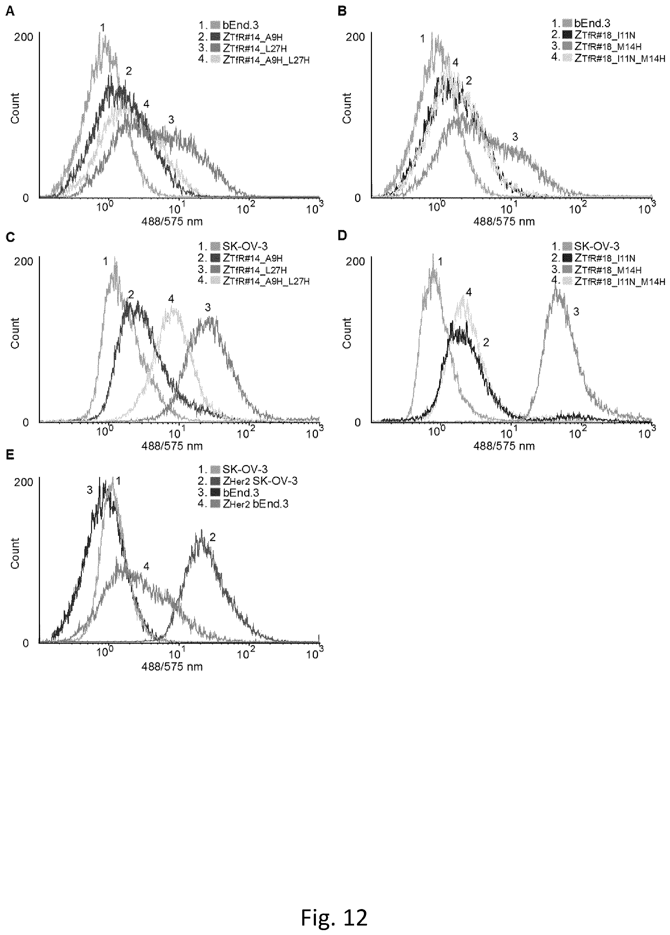

Figure 12. Flow cytometry analysis of FITC labelled $Z_{TfR\#14\_A9H}$, $Z_{TfR\#14\_L27H}$, $Z_{TfR\#14\_A9H\_L27H}$, $Z_{TfR\#18\_I11N}$, $Z_{TfR\#18\_M14H}$, $Z_{TfR\#18C11N\_M14H}$, and $Z_{HER2}$. **A-B)** bEnd.3 cells and **C-D)** SK-OV-3 cells incubated with 1 μM $Z_{TfR}$. **E)** bEnd.3 and SK-OV-3 cells incubated with HER2-specific control affibody ($Z_{HER2}$). bEnd.3 cells (black), bEnd.3 cells with $Z_{Her2}$ (dark grey), SK-OV-3 cells (light grey) and SK-OV-3 cells with $Z_{Her2}$ (grey).

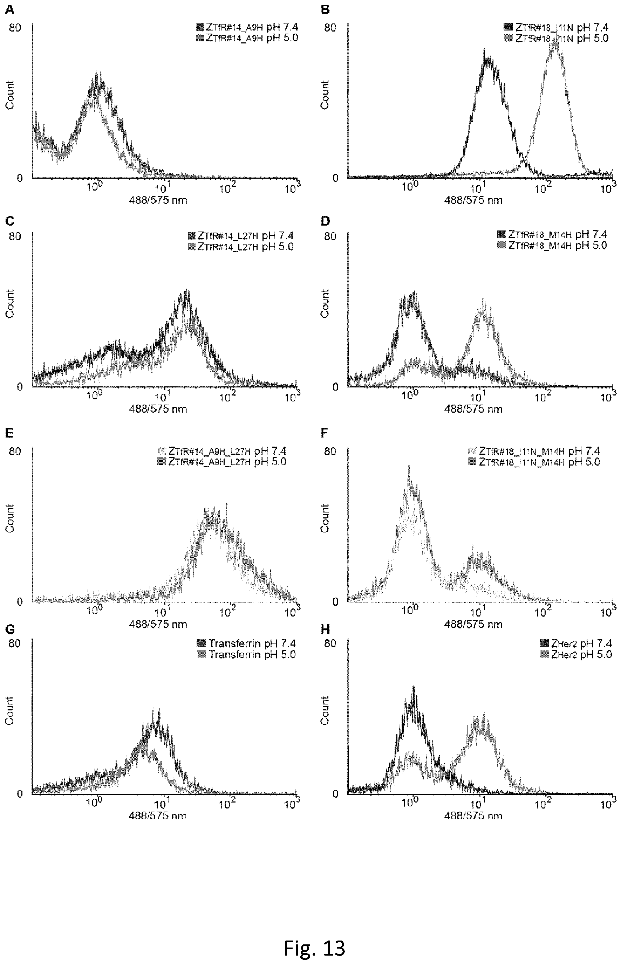

Figure 13. Flow cytometry on SK-OV-3 cells. Cells were incubated with $Z_{TfR}$, $Z_{HER2}$ or Tf-488 at pH 7.4 before cells were washed and divided into two samples for dissociation at pH 7.4 or 5.0 for 30 min. The samples are finally washed in pH 7.4 and analysed in the flow cytometer. The signal obtained after incubation at different pH for **(A)** $Z_{TfR\#14\_A9H}$, **(B)** $Z_{TfR\#18\_I11N}$, **(C)** $Z_{TfR\#14\_L27H}$, **(D)** $Z_{TfR\#18\_M14H}$, **(E)** $Z_{TfR\#14\_A9H\_L27H}$, **(F)** $Z_{TfR\#18\_I11N\_M14H}$, **(G)** Tf-488, and **(H)** $Z_{HER2}$, pH 7.4 (dark grey), and pH 5.0 (light grey).

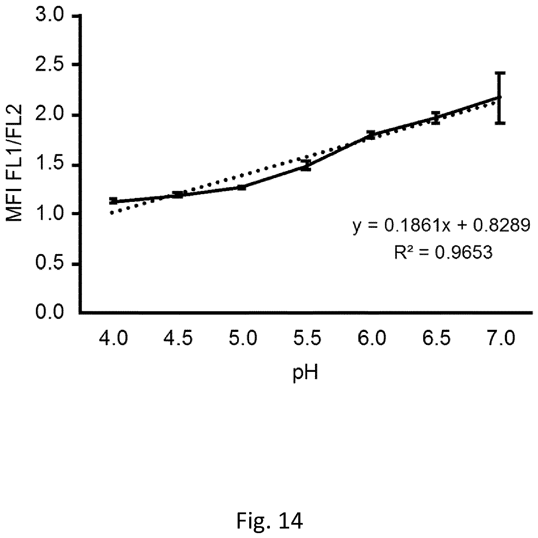

Figure 14. The standard curve from dextran-FITC mean fluorescent intensity (MFI) for FL1/FL2 ratio. The curve had a linear fit (dashed line) of 0.965 in regression value. Error bars for the triplicate standard points are given for each pH step.

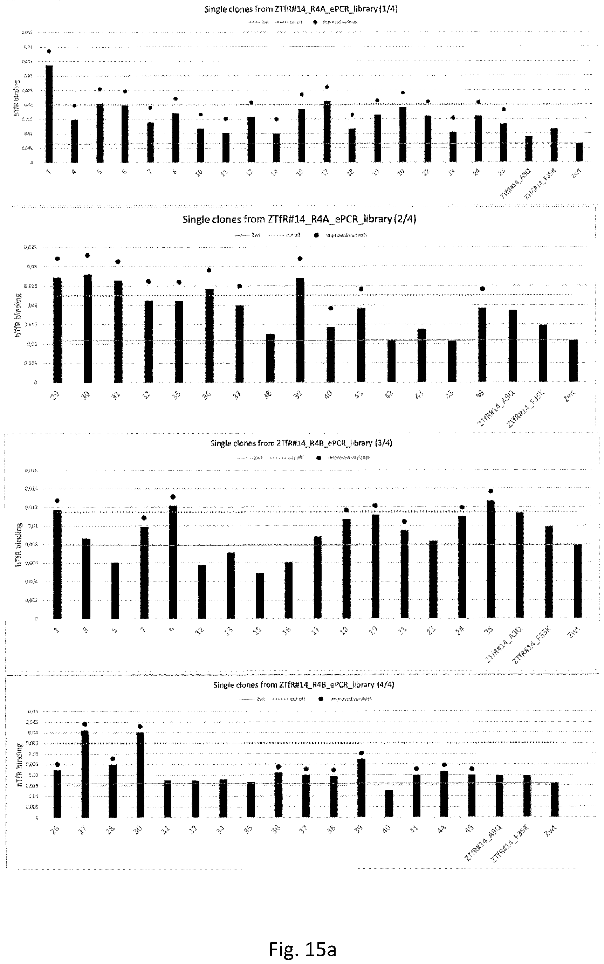

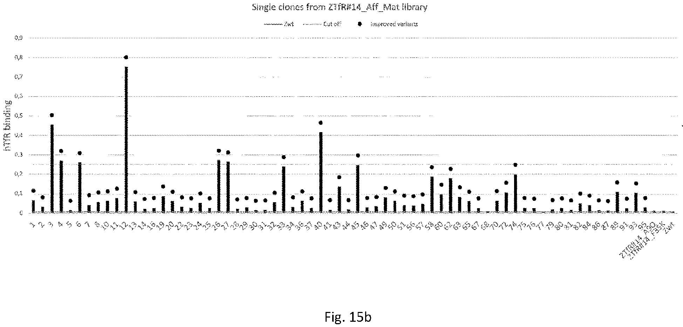

Figure 15. Mean fluorescence intensity (MFI) corresponding to hTfR binding from flow cytometry on *E. coli* cells displaying variants of $Z_{TfR\#14}$ in fusion to an albumin-binding domain. Cells were incubated with labelled human transferrin and fluorescently labelled HSA. Cells displaying first-generation variants of $Z_{TfR\#14}$ are included for comparison and cells displaying Zwt are included as negative control. Dots above bars indicate variants with retained or improved binding for hTfR. Dotted line (cut-off line) indicates threshold for variants with substantially improved binding for hTfR. Solid line indicates the signal from negative control (Zwt).

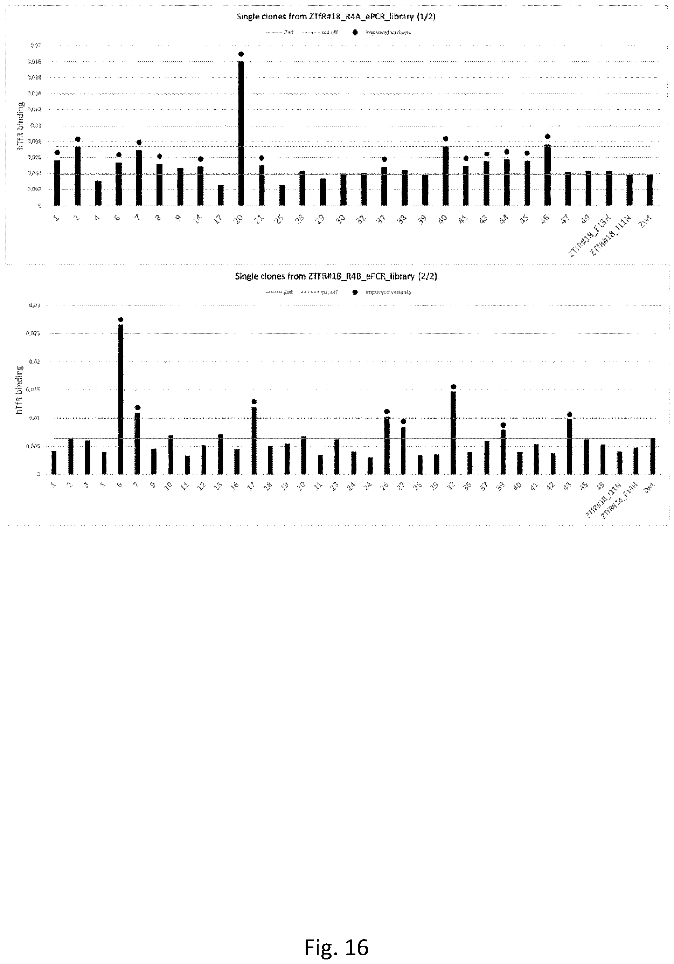

Figure 16. Mean fluorescence intensity (MFI) corresponding to hTfR binding from flow cytometry on *E. coli* cells displaying variants of $Z_{TfR\#18}$ in fusion to an albumin-binding domain. Cells were incubated with labelled human transferrin and fluorescently labelled HSA. Cells displaying first-generation variants of $Z_{TfR\#18}$ are included for comparison and cells displaying Zwt are included as negative control. Dots above bars indicate variants with retained or improved binding for hTfR. Dotted line (cut-off line) indicates threshold for variants with substantially improved binding for hTfR. Solid line indicates the signal from negative control (Zwt).

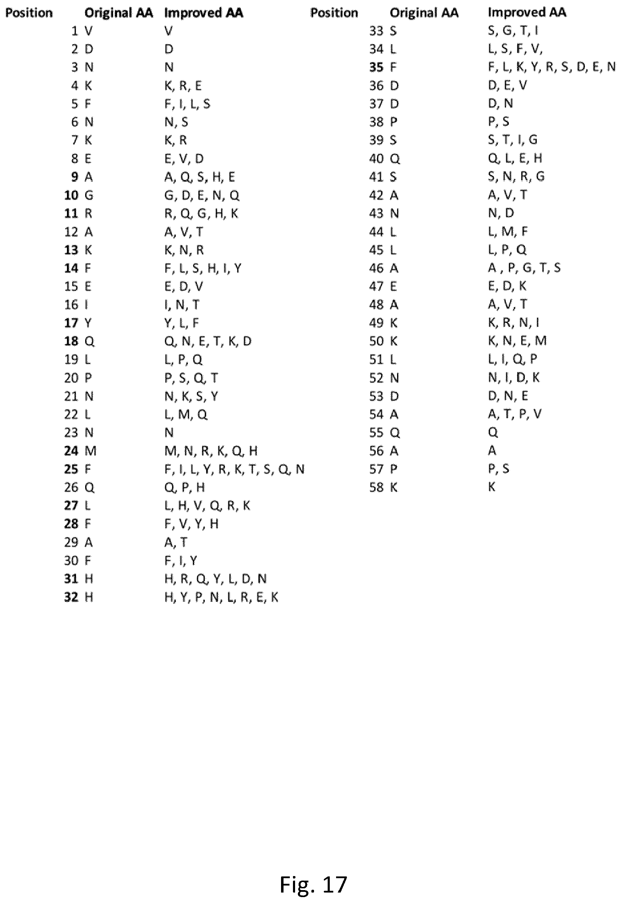

Figure 17. Amino acid distribution for $Z_{TfR\#14}$-based clones with improved or retained binding.

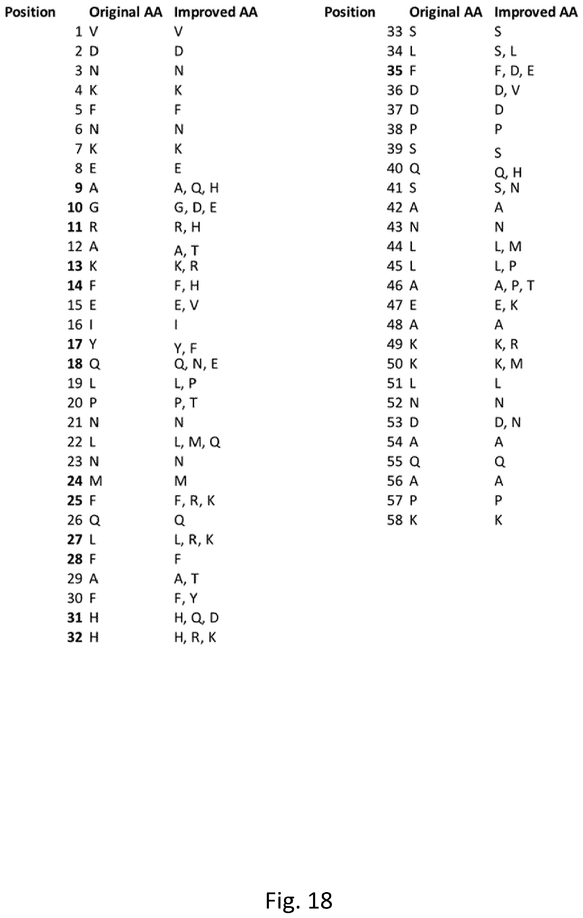

Figure 18. Preferred amino acid distribution based on clones with binding over the cut-off line shown in figure 15.

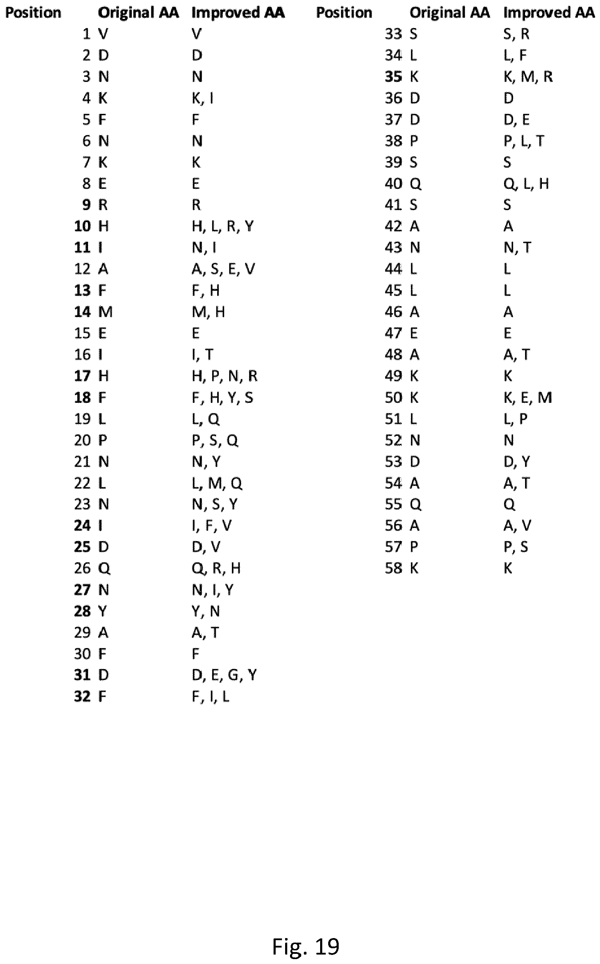

Figure 19. Amino acid distribution for $Z_{TfR\#18}$-based clones with improved or retained binding.

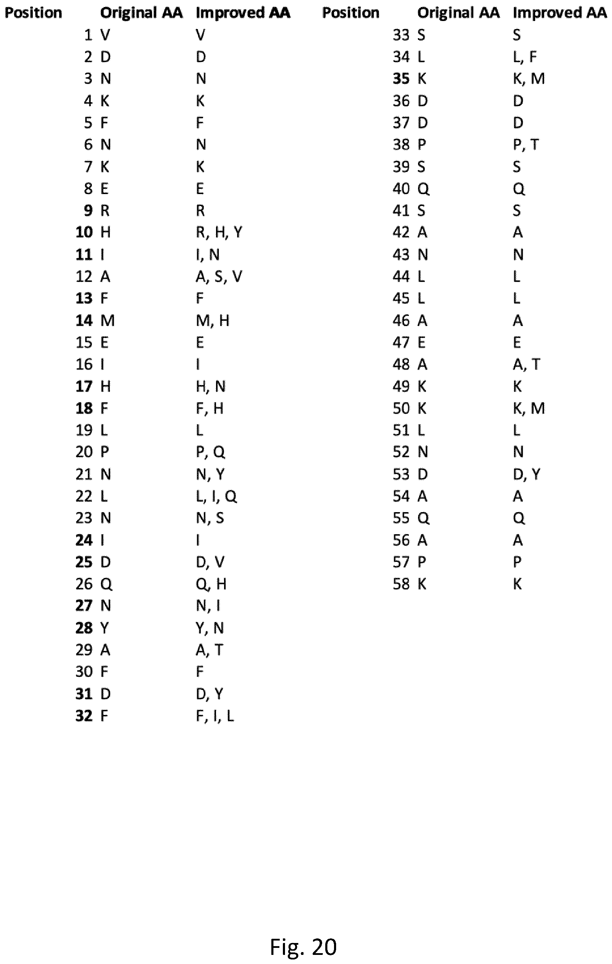

Figure 20. Preferred amino acid distribution based on clones with binding over the cut-off line shown in figure 16.

## DETAILED DESCRIPTION

The limited efficacy of many drugs, in particular CNS-targeted biological drugs, can be improved by an increased transportation across the BBB. The use of small alternative scaffolds (e.g. affibodies) as brain shuttles has an advantage over antibodies due to the faster biodistribution in the brain parenchyma after transcytosis. Smaller mass of the complex is expected to additionally decrease potential systemic toxic effects from long serum half-life in the periphery [18].

As shown herein, TfR-specific affibody molecules were selected using phage display technology. Nineteen candidates were selected from the output and the two most promising binders were further characterized *in vitro.* Affinity, binding kinetics, epitope, valency, and pH-dependent binding are examples of properties that have been suggested in the literature to be important for RMT via TfR [9,17,27,28]. Moreover, cross reactivity to TfR orthologues from model animals would facilitate future preclinical evaluation and clinical translation.

The two selected binders $Z_{TfR\#14}$ and $Z_{TfR\#18}$ were cross reactive for murine and human TfR and bound to an epitope that was non-overlapping with the site for Tf. Avoiding the binding site of Tf is probably important as the high concentration of Tf in blood would otherwise greatly limit available receptors for RMT. Furthermore, blocking the interaction between Tf and TfR has previously been connected to toxicity in mice [17]. Using multi-colour flow cytometry, simultaneous binding of Tf and respective affibody to cells was shown, at molar excess of either Tf or affibody. Thus, these affibodies will likely not be toxic due to decreased uptake of transferrin.

It has been hypothesized and demonstrated for some TfR-specific antibodies in *in vitro* models that pH-dependent binding is important for transcytosis [27]. The idea is in principle to mimic the natural pH dependency in the range between pH 5.0 to 7.4 that is part of the iron transport mechanism via Tf and TfR. It should however be noted that several TfR-specific brain shuttles without pH-dependent binding have been reported, and it is speculated that the importance of pH dependency is linked to TfR epitope and affinity [28,30]. The pH dependent binding can however be of importance since certain epitopes in combination with bivalent binders are believed to cross-link the receptor during endocytosis, directing it to lysosomal degradation. Still, release by pH or by fast off rates after endocytosis have probably potential to improve transcytosis [27]. $Z_{TfR\#14}$ and $Z_{TfR\#18}$ displayed different pH dependent behaviour, where $Z_{TfR\#14}$ was not affected by pH in the given range and $Z_{TfR\#18}$ had a slower off rate from the receptor with lower pH.

Single amino acid mutagenesis was performed on the two binders (i.e. $Z_{TfR\#14}$ and $Z_{TfR\#18}$), where histidine or the $Z_{wt}$ [31] amino acid was incorporated in the 14 previously randomised positions. The results from the mutagenesis study indicated the contribution of individual residues to the interaction with TfR. Moreover, a few mutants showed increased binding to TfR and two single amino acid mutants for each affibody were analyzed further. The two mutations were also combined in double mutants to evaluate potential additive effects. The new variants ($Z_{TfR\#14\_A9H}$, $Z_{TfR\#14\_L27H}$, $Z_{TfR\#i4\_A9H\_L27H}$, $Z_{TfR\#18-I11N}$, $Z_{TfR\#18\_M14H}$, and $Z_{TfR\#18I\_11N\_M14H}$) were site-specifically conjugated to FITC and showed binding to both SK-OV-3 and brain endothelial bEnd.3 cells in flow cytometry. Internalization was analysed using a fluorescence assay, exploiting the pH-dependency of FITC and indicating that the binders were located in a cell compartment with a pH of around 5-6 for the $Z_{TfR\#18}$-derived variants and around 6-7 for the $Z_{TfR\#18}$-derived variants. The results suggest that the $Z_{TfR\#18}$-derived variants are more efficiently internalized, although a pH of around 5 might indicate that it is partly directed to lysosomes, which is undesired. Finally, the six affibodies were studied with a method where nanofibrillar membranes of recombinant silk seeded with brain endothelial cells are used as a simplified BBB model to assess active transcytosis. The results showed particularly high apparent permeability for three of the TfR-binders compared to a negative control.

A further mutagenesis study of $Z_{TfR\#14}$ and $Z_{TfR\#18}$ was performed to evaluate the binding contribution of amino acids and how substituting to other amino acids affects binding to TfR. Error-prone PCR was used to introduce random mutations in the genes encoding for Z#14 and Z#18, respectively. In addition, a site-directed mutagenesis library was constructed for $Z_{TfR\#14}$, limiting the mutations to the previously 14 randomized positions in helices 1 and 2. Oligos encoding the mutated affibody genes were subcloned to the *E. coli* display vector pBad2.2 [21], and transformed to *E. cloni* EXPRESS BL21(DE3) electrocompetent cells *E. coli* (Lucigen, Wisconsin, USA) by electroporation. Colonies were sequence-verified by sanger sequencing (Eurofins Genomics, Ebersberg, Germany). Transformed *E. coli* were grown in LB media at 37 °C until $OD_{600}$ reached 0.5 AU before inducing with arabinose (0.6%) and incubation at 25 °C for 16 h protein expression. Cells expressing affibody molecules on the surface were incubated in biotinylated hTfR (#H82E5, Acro Biosystems, Newark, DE, USA) for 45 min. After washing with PBS with 0.01% Pluronic F108 NF surfactant (PBSP), cells were incubated on ice, with streptavidin phycoerythrin conjugate (SA-PE; Thermo Scientific, Waltham, MA, USA) and HSA-AF647 (in-house labelled). After washing, binding populations were isolated by FACS using a CytoFLEX SRT cell sorter (Beckman Coulter, Brea, CA, USA). After FACS, individual variants from the sorting output displayed on *E. coli* were analyzed for TfR-binding on a CytoFLEX S flow cytometer (Beckman Coulter, Brea, CA, USA) and data analysed in Kaluza (Version 2.1, Beckman Coulter, Brea, CA, USA). Sanger sequencing was used to identify variants showing improved or retained TfR binding.

Accordingly, there is provided, as a first aspect of the present disclosure, a transferrin receptor 1-binding polypeptide comprising the amino acid sequence

$X_9 X_{10} X_{11} X_{12} X_{13} X_{14} X_{15} X_{16} X_{17} X_{18} X_{19} X_{20} X_{21} X_{22} N X_{24} X_{25} X_{26} X_{27} X_{28} X_{29} X_{30} X_{31} X_{32} X_{33} X_{34} X_{35}$ (SEQ ID NO:1) wherein

$X_9$ is A, Q, S, H or E,
$X_{10}$ is G, D, E, N or Q,
$X_{11}$ is R, Q, G, H or K,
$X_{12}$ is A, V or T,
$X_{13}$ is K, N or R,
$X_{14}$ is F, L, S, H, I or Y,
$X_{15}$ is E, D or V,
$X_{16}$ is I, N or T,
$X_{17}$ is Y, L or F,
$X_{18}$ is Q, N, E, T, K or D,
$X_{19}$ is L, P or Q,
$X_{20}$ is P, S, Q or T,
$X_{21}$ is N, K, SorY,
$X_{22}$ is L, M or Q,
$X_{24}$ is M, N, R, K, Q or H,
$X_{25}$ is F, I, L, Y, R, K, T, S, Q or N,
$X_{26}$ is Q, P or H,
$X_{27}$ is L, H, V, Q, R or K,
$X_{28}$ is F, V, Y or H,
$X_{29}$ is A or T,
$X_{30}$ is F, I or Y,
$X_{31}$ is H, R, Q, Y, L, D or N,
$X_{32}$ is H, Y, P, N, L, R, E or K,
$X_{33}$ is S, G, T or I,
$X_{34}$ is L, S, F or V and
$X_{35}$ is F, L, K, Y, R, S, D, E, N.

The alternatives for the "X" positions are based on figure 17 discussed in the examples section below. Positions 9-35 are known to be of particular relevance for the binding capacity.

In a preferred embodiment of the polypeptide of the first aspect

$X_9$ is A, Q, S, H or E,
$X_{11}$ is R, H or K,
$X_{12}$ is A, V or T,
$X_{13}$ is K or R,
$X_{14}$ is F, H, I or Y,
$X_{15}$ is E or V,
$X_{16}$ is I,
$X_{17}$ is Y, L or F,
$X_{18}$ is Q, N, E, T, K or D,
$X_{19}$ is L or P,

$X_{20}$ is P, S or T,
$X_{21}$ is N,
$X_{22}$ is L, M or Q,
$X_{25}$ is F, I, L, R, K, T, S, Q or N,
$X_{26}$ is Q,
$X_{27}$ is L, H, Q, R or K,
$X_{30}$ is F or Y,
$X_{31}$ is H, Q, D or N,
$X_{33}$ is S or G,
$X_{34}$ is L or S and/or
$X_{35}$ is F, L, K, Y, S, D, E or N.

In a more preferred embodiment of the polypeptide of the first aspect

$X_9$ is A, Q or H,
$X_{10}$ is G, D or E,
$X_{11}$ is R or H,
$X_{12}$ is A or T,
$X_{13}$ is K or R,
$X_{14}$ is F or H,
$X_{15}$ is E or V,
$X_{16}$ is I,
$X_{17}$ is Y or F,
$X_{18}$ is Q, N or E,
$X_{19}$ is L or P,
$X_{20}$ is P or T,
$X_{21}$ is N,
$X_{24}$ is M,
$X_{25}$ is F, I, R or K, preferably F, R or K
$X_{26}$ is Q,
$X_{27}$ is L, R, H or K,
$X_{28}$ is F,
$X_{30}$ is F or Y,
$X_{31}$ is H, Q or D,
$X_{32}$ is H, R or K,
$X_{33}$ is S,
$X_{34}$ is L or S and/or
$X_{35}$ is F, D or E.

These preferred amino acids are based on figure 18 and the experiment behind it.
In the polypeptide of the first aspect, $X_{27}$ is preferably H when $X_9$ is H.
In one embodiment (based on figure 17), the polypeptide of the first aspect further comprises the amino acid sequence $X_4X_5X_6X_7X_8$ at the N terminus of SEQ ID NO:1, which means that is comprises the following sequence:

$$X_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}$$

$$X_{32}X_{33}X_{34}X_{35} \text{ (SEQ ID NO:22)}.$$

In this embodiment

$X_4$ is K, R or E, preferably K,
$X_5$ is F, I, L or S, preferably F or S, more preferably F,
$X_6$ is N or S, preferably N,
$X_7$ is K or R, preferably K, and
$X_8$ is E, V or D, preferably E or V, more preferably E.

The preferred amino acids of the embodiment are based on figure 18 and the experiment behind it.

In one embodiment, the N terminus of SEQ ID NO:1 is further extended by the sequence VDN (see figure 17 and 18). According to this embodiment, the polypeptide of the first aspect thus comprises the following sequence:

$$VDNX_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}$$

$$X_{31}X_{32}X_{33}X_{34}X_{35} \text{ (SEQ ID NO:23)}.$$

In an alternative or complementary embodiment (also based on figure 17), the polypeptide of the first aspect further comprises the amino acid sequence $X_{36}X_{37}X_{38}X_{39}X_{40}$ at the C terminus of SEQ ID NO:1, which means that it comprises the sequence

$$X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}X_{32}X_{33}X_{34}X_{35}$$

$$X_{36}X_{37}X_{38}X_{39}X_{40} \text{ (SEQ ID NO:24)}.$$

In this embodiment

$X_{36}$ is D, E or V, preferably D or V,
$X_{37}$ is D or N, preferably D,
$X_{38}$ is P or S, preferably P,
$X_{39}$ is S, T, I or G, preferably S or T, more preferably S, and
$X_{40}$ is Q, L, E or H, preferably Q, E or H, more preferably Q or H.

The preferred amino acids of the embodiment are based on figure 18 and the experiment behind it.
In one embodiment, the C terminus of SEQ ID NO:1 is further extended by the sequence $X_{41}X_{42}X_{43}X_{44}X_{45}X_{46}X_{47}X_{48}X_{49}X_{50}X_{51}X_{52}X_{53}X_{54}$ (see figure 17). According to this embodiment, the polypeptide of the first aspect thus comprises the following sequence:

$$X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}X_{32}X_{33}X_{34}X_{35}$$

$$X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54} \text{ (SEQ ID NO:25)}.$$

In this embodiment

$X_{41}$ is S, N, R or G, preferably S or N,
$X_{42}$ is A, V or T, preferably A,
$X_{43}$ is N or D, preferably N,
$X_{44}$ is L, M or F, preferably L or M,
$X_{45}$ is L, P or Q, preferably L or P,
$X_{46}$ is A, P, G, T or S, preferably A, P or T,
$X_{47}$ is E, D or K, preferably E or K,
$X_{48}$ is A, V or T, preferably A,
$X_{49}$ is K, R, N or I, preferably K or R,
$X_{50}$ is K, N, E or M, preferably K or M,
$X_{51}$ is L, I, Q or P, preferably L,
$X_{52}$ is N, I, D or K, preferably N,
$X_{53}$ is D, N or E, preferably D or N, and
$X_{54}$ is A, T, P or V, preferably A.

The preferred amino acids of the embodiment are based on figure 18 and the experiment behind it.
The C terminus of SEQ ID NO:1 may be still further extended by the sequence $QAX_{57}K$, which results in the following sequence:

$$X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}X_{32}X_{33}X_{34}X_{35}$$

$$X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}QAX_{57}K \text{ (SEQ ID NO:26).}$$

In such case, $X_{57}$ is P or S, preferably P (see figures 17 and 18).

It follows from the above, that the polypeptide of the first aspect may comprise any of the following sequences:

$$X_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}$$

$$X_{32}X_{33}X_{34}X_{35} X_{36}X_{37}X_{38}X_{39}X_{40} \text{ (SEQ ID NO:27);}$$

$$VDNX_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}$$

$$X_{31}X_{32}X_{33}X_{34}X_{35} X_{36}X_{37}X_{38}X_{39}X_{40} \text{ (SEQ ID NO:28);}$$

$$X_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}$$

$$X_{32}X_{33}X_{34}X_{35} X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54} \text{ (SEQ ID NO:29);}$$

$$VDNX_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}$$

$$X_{31}X_{32}X_{33}X_{34}X_{35} X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54} \text{ (SEQ ID NO:30);}$$

$$X_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}$$

$$X_{32}X_{33}X_{34}X_{35} X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}QAX_{57}K \text{ (SEQ ID NO:31); and}$$

$$VDNX_4X_5X_6X_7X_8X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}$$

$$X_{31}X_{32}X_{33}X_{34}X_{35}X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}QAX_{57}$$

$$K \text{ (SEQ ID NO:32).}$$

In one embodiment, the polypeptide of the first aspect comprises or consists of the sequence SEQ ID NO:7 (see figure 1), optionally having a L27H mutation (see e.g. figure 6). In addition to the L27H mutation, there can be a A9H mutation in SEQ ID NO:7 (see e.g. figure 6).

As a second aspect of the present disclosure, there is provided a transferrin receptor 1-binding polypeptide comprising the amino acid sequence

$RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}X_{32}X_{33}X_{34}X_{35}$ (SEQ ID NO:2) wherein

$X_{10}$ is H, L, R or Y,
$X_{11}$ is N or I,

$X_{12}$ is A, S, E or V,
$X_{13}$ is F or H,
$X_{14}$ is M or H,
$X_{16}$ is I or T,
$X_{17}$ is H, P, N or R,
$X_{18}$ is F, H, Y or S,
$X_{19}$ is L or Q,
$X_{20}$ is P, S or Q,
$X_{21}$ is N or Y,
$X_{22}$ is L, M, I or Q,
$X_{23}$ is N, S or Y,
$X_{24}$ is I, F or V,
$X_{25}$ is D or V,
$X_{26}$ is Q, R or H,
$X_{27}$ is N, I or Y,
$X_{28}$ is Y or N,
$X_{29}$ is A or T,
$X_{31}$ is D, E, G or Y,
$X_{32}$ is F, I or L,
$X_{33}$ is S or R,
$X_{34}$ is L or F and
$X_{35}$ is K, M or R.

The alternatives for the "X" positions are based on figure 19 discussed in the examples section below. Positions 9-35 are known to be of particular relevance for the binding capacity.
In a preferred embodiment of the polypeptide of the second aspect

$X_{10}$ is H, R or Y,
$X_{12}$ is A, S or V,
$X_{13}$ is F,
$X_{16}$ is I,
$X_{17}$ is H or N,
$X_{18}$ is F or H,
$X_{19}$ is L,
$X_{20}$ is P or Q,
$X_{21}$ is N or Y,
$X_{22}$ is L, I or Q,
$X_{23}$ is N or S,
$X_{24}$ is I,
$X_{25}$ is D or V,
$X_{26}$ is Q or H,
$X_{27}$ is N or I,
$X_{28}$ is Y or N,
$X_{29}$ is A or T,
$X_{31}$ is D or Y,
$X_{32}$ is F, I or L,
$X_{33}$ is S,
$X_{34}$ is L or F and/or
$X_{35}$ is K or M.

This preferred embodiment is based on figure 20 and the experiment behind it.
In an embodiment of the second aspect (based on figure 19), the polypeptide further comprising the amino acid sequence $X_4 X_5 X_6 X_7 X_8$ at the N terminus of SEQ ID NO:2, which means that it comprises the following sequence

$X_4 X_5 X_6 X_7 X_8 R X_{10} X_{11} X_{12} X_{13} X_{14} E X_{16} X_{17} X_{18} X_{19} X_{20} X_{21} X_{22} X_{23} X_{24} X_{25} X_{26} X_{27} X_{28} X_{29} F X_{31} X_{32}$

$X_{33} X_{34} X_{35}$ (SEQ ID NO:33).

In this embodiment

$X_4$ is K or I, preferably K (see figure 20),
$X_5$ is F,
$X_6$ is N,
$X_7$ is K, and
$X_8$ is E.

The N terminus of SEQ ID NO: 2 may be further extended by the sequence VDN (see figure 19). In such case, the polypeptide of the second aspect comprises the sequence

$$VDNX_4X_5X_6X_7X_8RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}$$

$$X_{32}X_{33}X_{34}X_{35} \text{ (SEQ ID NO:34)}.$$

In an alternative or complementary embodiment (based on figure 19), the polypeptide of the second aspect further comprises the amino acid sequence $X_{36}X_{37}X_{38}X_{39}X_{40}$ at the C terminus of SEQ ID NO:2, which means that it comprises the following sequence

$$RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}X_{32}X_{33}X_{34}X_{35}X_{36}$$

$$X_{37}X_{38}X_{39}X_{40} \text{ (SEQ ID NO:35)}.$$

In this embodiment

$X_{36}$ is D,
$X_{37}$ is D or E, preferably D,
$X_{38}$ is P, L or T, preferably P or T,
$X_{39}$ is S, and
$X_{40}$ is Q, L or H, preferably Q.

The preferred amino acids of the embodiment are based on figure 20 and the experiment behind it.
The C terminus of SEQ ID NO:2 may be further extended by the sequence $X_{41}X_{42}X_{43}X_{44}X_{45}X_{46}X_{47}X_{48}X_{49}X_{50}X_{51}X_{52}X_{53}X_{54}$. In such an embodiment (which is based on figure 19), the polypeptide of the second aspect comprises the following sequence

$$RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}X_{32}X_{33}X_{34}X_{35}X_{36}$$

$$X_{37}X_{38}X_{39}X_{40}X_{41}X_{42}X_{43}X_{44}X_{45}X_{46}X_{47}X_{48}X_{49}X_{50}X_{51}X_{52}X_{53}X_{54} \text{ (SEQ ID NO:36)}.$$

In this embodiment

$X_{41}$ is S,
$X_{42}$ is A,
$X_{43}$ is N or T, preferably N,
$X_{44}$ is L,
$X_{45}$ is L,
$X_{46}$ is A,
$X_{47}$ is E,
$X_{48}$ is A or T,
$X_{49}$ is K,
$X_{50}$ is K, E or M, preferably K or M,
$X_{51}$ is L or P, preferably L,
$X_{52}$ is N,
$X_{53}$ is D or Y, and

$X_{54}$ is A or T, preferably A.

The preferred amino acids of the embodiment are based on figure 20 and the experiment behind it.

The C terminus of SEQ ID NO:2 may be still further extended by the sequence $QX_{56}X_{57}K$ (see figure 19). In such an embodiment, the polypeptide of the second aspect thus comprises the following sequence

$$RX_{10}X_{11}X_{12}X_{13}X_{14}EX_{16}X_{17}X_{18}X_{19}X_{20}X_{21}X_{22}X_{23}X_{24}X_{25}X_{26}X_{27}X_{28}X_{29}FX_{31}X_{32}X_{33}X_{34}X_{35}X_{36}$$

$$X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}QX_{56}X_{57}K\ (\text{SEQ ID}$$

NO:37).

In this embodiment, $X_{56}$ is A or V, preferably A, and $X_{57}$ is P or S, preferably P. The preferences are based on figure 20 and the experiment behind it.

In one embodiment, the polypeptide of the second aspect comprises or consists of the sequence SEQ ID NO:8 (see figure 1), optionally having a I11N mutation and/or a M14H mutation (see e.g. figure 6).

As a third aspect of the present disclosure, there is provide a fusion protein or conjugate comprising:

- a first part comprising a polypeptide according to the first or the second aspect; and
- a second part having a therapeutic activity.

Said second part may for example comprise an agent selected from the group consisting of inhibitors of neurotransmitter degradation, neurotransmitters, acetylcholinesterase inhibitors, NMDA receptor antagonists, TNF inhibitors, antihistamines, anti-viral agents, alpha-secretase activators, inhibitors of beta- or gamma-secretase, inhibitors of α-synuclein aggregation, inhibitors of tau aggregation, calcium channel blockers, compounds effective against oxidative stress, anti-apoptotic compounds, metal chelators, inhibitors of DNA repair such as pirenzepin and metabolites, attractants for Aβ clearing/depleting cellular components, inhibitors of N-terminal truncated Aβ including pyroglutamated Aβ3-42, anti-inflammatory molecules, atypical antispychotics such as clozapine, ziprasidone, risperidone, aripiprazole and olanzapine, cholinesterase inhibitors (ChEIs) such as tacrine, rivastigmine, donepezil and galantamine, M1 agonists and other drugs including any amyloid or tau modifying drug and nutritive supplements such as vitamin B12, cysteine, acetylcholine precursor, lecithin, choline, *Ginkgo biloba,* acetyl-L-carnitine, idebenone, propentofylline, and xanthine derivatives.

In one embodiment, the therapeutic activity is based on binding to a target selected from the group consisting of amyloid β, Tau, apolipoprotein E (ApoE), beta-secretase 1 (BACE1, [4]), gamma-secretase, complement factor C1s, presenilin 1, presenilin 2, nicastrin, alpha-synuclein, sortilin, progranulin, prosaposin and Bri. As known to the skilled person, these are targets associated with neurological disorders.

Another option for the therapeutic activity is binding to a target selected from the group consisting of ACE, ICA1L, MAP1S, SLC20A2 and TOM1L2. In particular, these targets are associated with treatment or prevention of Alzheimer's disease [32].

In a specific embodiment of the third aspect, the polypeptide of the first or the second aspect is fused to $Z_{SYM73}$ (discussed above) or a related amyloid β binder. Here, a related amyloid β binder is defined as a binder comprising a first moiety having a sequence corresponding to amino acid residues 4-25 in any one of SEQ ID NO: 9, 10, 11, 12, 13, 14, 15 and 16 and a second moiety having a sequence corresponding to amino acid residues 69-90 in SEQ ID NO: 9 or amino acid residues 64-85 in any one of SEQ ID NO: 10, 11, 12, 13, 14, 15 and 16 (see WO2016/131987).

SEQ ID NO:9 herein is SEQ ID NO:18 in WO2016/131987.

SEQ ID NO:10 herein is SEQ ID NO:28 in WO2016/131987.

SEQ ID NO:11 herein is SEQ ID NO:35 in WO2016/131987.

SEQ ID NO:12 herein is SEQ ID NO:49 in WO2016/131987.

SEQ ID NO:13 herein is SEQ ID NO:50 in WO2016/131987.

SEQ ID NO:14 herein is SEQ ID NO:70 in WO2016/131987.

SEQ ID NO:15 herein is SEQ ID NO:84 in WO2016/131987.

SEQ ID NO:16 herein is SEQ ID NO:95 in WO2016/131987.

In another specific embodiment of the third aspect, the polypeptide of the first or the second aspect is fused to an amyloid β binder comprising an amino acid sequence selected from i) and ii), wherein:

i) is SEQ ID NO:17 - [LINK] - SEQ ID NO:18;
ii) is SEQ ID NO:19 - [LINK] - SEQ ID NO:20; and [LINK] is a linker. The linker may for example be the amino acid sequence

AEAKKLNDAQAPASSSSGSSSSGRASAGGE (SEQ ID NO: 21)

Here, i) and ii) are based on Sq$_{A\beta22}$ and SqA$_{\beta23}$ in Hjelm 2023 [24].

Yet other options for the therapeutic activity is binding to a target selected from the group consisting of CD38, DGKQ, GPNMB and SEC23IP. In particular, these targets are associated with treatment or prevention of Parkinson's disease [32].

The therapeutic activity may also be binding to a target selected from the group consisting of DHRS11, FAM120B, SHMT1 and TSFM. In particular, these targets are associated with treatment or prevention of multiple sclerosis [32].

In one embodiment, the therapeutic activity is used in a brain cancer treatment activity. In such an embodiment, the treatment activity may be based on binding to a tumour-associated antigen, EGFR, a CMV antigen, NKG2DL, ERBB2, IL13RA2 or WT1. For these targets, the binder of the second part is typically a biological agent [33], such as a polypeptide or protein, which may form a fusion protein with the first part.

In case of a construct, the brain cancer treatment activity may be based on binding to DNA, VEGFA, PDCD1, NR1I2, KDR, PTGS2, PPARG, EGFR, MTOR, PTGS1, TUBB1, ACAT1, FLT1, KIT, PDGFRB, FLT4, HRH2, TOP1, PDGFRA, TOP1MT, TOP2A, HDAC2, FGFR1, TACSTD2, TYMS, CDK4, CDK6, FLT3, STMN4, BRAF, PIK3CA, RET, CSF2RA, CSF2RB, FGFR2, HDAC1, IL3RA, PRG2, RRM1, SDC2, DRD1 or FGFR3 [33].

In an embodiment of the third aspect of the present disclosure, the fusion protein or conjugate further comprises:

- a third part comprising a half-life-extending region, such as an Fc-binding region or an albumin-binding region (ABR).

The ABR may for example be one of the albumin binding domains (ABDs) discussed below. Various half-life-extending strategies for polypeptides/proteins are described in a review article by Kontermann [34].

As a configuration of the third aspect, there is provided a fusion protein or conjugate comprising:

- a first part comprising a polypeptide according to the first or the second aspect; and
- a second part binding a diagnostic target.

Such a fusion protein or conjugate may enable visualization of the presence and/or abundance of the diagnostic target in the brain region by molecular imaging (MI) techniques including positron emission tomography (PET), singlephoton emission computed tomography (SPECT), near-infrared fluorescence (NIRF), bioluminescence imaging (BLI) and magnetic resonance imaging (MRI).

As a fourth aspect of the present disclosure, there is provided a polypeptide according to the first or the second aspect or fusion protein or conjugate according to the third aspect for use as a medicament.

As a fifth aspect of the present disclosure, there is provided a pharmaceutical composition comprising the polypeptide according to the first or the second aspect or fusion protein or conjugate according to the third aspect. The composition of the fifth aspect is preferably adapted for intravenous or subcutaneous injection.

As a sixth aspect of the present disclosure, there is provided a polypeptide, fusion protein, conjugate or pharmaceutical composition of any one of the preceding aspects for use in a method of treatment of a disorder.

As a seventh aspect of the present disclosure, there is provided a method of treatment or prevention of a disorder in a subject, said method comprising administration of a polypeptide, fusion protein, conjugate or pharmaceutical composition of any one of the preceding aspects.

The disorder of the sixth or seventh aspect is preferably a neurological disorder, more preferably Parkinson's disease or dementia. Said dementia is preferably Lewy body dementia or Alzheimer's disease.

As discussed above, the disorder may also be MS or brain cancer. Another disorder of interest is glaucoma.

The method of the sixth or seventh aspect typically comprises injection, such as intravenous or subcutaneous injection, of the polypeptide, fusion protein, conjugate or pharmaceutical composition.

The subject of the method of the sixth or seventh aspect is preferably human.

**EXAMPLES**

**Methods and material**

Protein labelling

Biotinylation of recombinant human and mouse transferrin receptor-1 (TfR; Sino Biological Inc, Bejing, China) was performed using a Biotin-XX Microscale Protein Labeling Kit (Invitrogen, Waltham, MA, USA) according to supplier's recommendations. The concentration of the proteins was determined using absorbance.

Phage display selections

A combinatorial phage library of the Z domain with randomisation in 14 positions, was prepared essentially as described previously [35] (figure 1). Selection and amplification were performed in phosphate-buffered saline with tween (PBST; 0.1% Tween-20) with bovine serum albumin (BSA; Saveen&Werner, Limhamn, Sweden) (PBSTB; 3 w/v% BSA) at room temperature, as described previously [22]. Prior to the first cycle of bio-panning, a negative selection of the library was conducted by incubating the library with streptavidin-coated magnetic beads. The remaining phage particles were panned against recombinant transferrin receptor for 1 h in five cycles with increasing number of washes over the cycles. Different strategies for panning of binders were implemented, based on recombinant human transferrin receptor, recombinant murine transferrin receptor or cross-selection strategies. In the tracks where the target was kept constant, the concentration of the receptor was decreased from 100 nM in the first panning cycle to 12.5 nM in cycle five. In the cross-selection strategies, human and mouse receptor targets were altered in the five panning cycles, with target concentration lowered from 100 nM in the first two cycles to 40 nM in the last cycle (figure 7). Selections were followed by DNA sequencing (Microsynth AG, Balgach, Switzerland) of 174 randomly picked colonies.

Production of recombinant affibody molecules in *E. coli*

The affibody molecules selected for further characterization were produced with either a C-terminal hexa-histidine ($His_6$) tag, or an N-terminal hexa-histidine ($His_6$) in combination with a C-terminal albumin binding domain (ABD) [36] for purification and characterization strategies. For $His_6$-tagged proteins, the genetic sequences were subcloned into the pET26b(+) vector, introducing a C-terminal $His_6$-tag and yielding the protein constructs $[Z_{TfR}]$-$His_6$. For ABD-fused proteins, the affibody genes were cloned into a pT7 vector introducing a C-terminal $ABD_{035}$ molecule [36] yielding the final proteins $His_6$-$[Z_{TfR}]$-$(G_4S)_3$-$ABD_{035}$. Transformation to *Escherichia coli* BL21STAR (DE3) cells (Thermo Scientific, Waltham, MA, USA) was done by heat shock with the expression vectors, and colonies were sequence-verified by sanger sequencing (Microsynth AG, Balgach, Switzerland). For protein expression, cells were cultivated in tryptic soy broth with yeast extract (TSBY) media (Merck KGaA, Darmstadt, Germany) with 50 $\mu$g/mL kanamycin at 37 °C with 150 rpm shaking. At $OD_{600}$ of approximately 0.7 AU, protein expression was induced by addition of Isopropyl $\beta$-D-1-thiogalactopyranoside (IPTG; Chemtronica, Stockholm, Sweden) to a final concentration of 1 mM. The cultures were incubated for approximately 18 h at 25 °C prior to harvest and cells were lyzed by sonication with a Vibra-Cell VCX 130 sonicator (Sonics, Newtown, CT, USA). The affibody molecules were purified by immobilized metal affinity chromatography (IMAC) using a HisPur™ Cobalt resin (Thermo Fisher Scientific, Waltham, MA, USA) with running buffer (47 mM $Na_2HPO_4$, 3 mM $NaH_2PO_4$, 300 mM NaCl, 15 mM imidazole, pH 7.4), and elution buffer supplemented with 150 mM imidazole. All eluted proteins were buffer exchanged on PD-10 columns (Cytiva, Marlborough, MA, USA) to phosphate-buffered saline (PBS). The protein concentrations were determined by a Pierce™ BCA Protein Assay Kit as by manufacturer's instructions (Thermo Scientific, Waltham, MA, USA). The molecular weight and purity of the proteins was confirmed by sodium dodecyl sulphate-polyacrylamide gel electrophoresis (SDS-PAGE) (NuPAGE Bis-Tris 4-12 %, Invitrogen, Waltham, MA, USA) and MALDI mass spectrometry (MS) analysis using SCIEX 4200 MALDI-TOF instrument (SCIEX, Framinghamn, MA, USA).

TfR-positive cell line cultivation

Three cell lines were used to test for binding to TfR. The murine TfR-positive BBB cell line bEnd.3 (ATCC, United States) was cultivated in DMEM high glucose media (Thermo Fisher, Waltham, MA, USA) supplemented with 10% fetal bovine serum (FBS; Sigma-Aldrich/Merck KGaA, Damstadt, Germany) at 37 °C with 5% $CO_2$ until 80% confluency. The human TfR-positive SK-OV-3 (ATCC, Manassas, VA, USA) cell line was cultivated in McCoy media (Thermo Fisher, Waltham, MA, USA) supplemented with 10% FBS (Sigma-Aldrich/Merck KGaA, Damstadt, Germany) under the same conditions. The human TfR-positive hCMEC/D3 (#SCC066, Merck KGaA, Darmstadt, Germany) endothelial brain cell line was cultivated in EndoGRO™-MV Complete Media Kit (#SCME004, Merck KGaA, Damstadt, Germany) supplemented with 1 ng × mL$^{-1}$ fibroblast growth factor-2 (FGF-2) (#GF003, Merck KGaA, Damstadt, Germany) in T75 flasks pre-coated with Collagen Type I, Rat Tail (#08-115, Merck KGaA, Damstadt, Germany) in an atmosphere of 5% $CO_2$ at 37 °C as by manufacturer's instructions. To detach cells, TrypLE Express Gibco™ (Thermo Fisher, Waltham, MA, USA) was added to cells in incubator for 3-5 minutes. Recovered and washed cells were resuspended in ice-cold PBS supplemented with 1 w/v% BSA (PBSB) (Saveen&Werner, Limhamn, Sweden).

Flow cytometry analysis of $Z_{TfR}$-binding to murine and human cells

A dilution series of each affibody in $His_6$-$[Z_{TfR}]$-ABD format was incubated with 100,000 human TfR-positive SK-OV-3 cells, respectively, for 45 min, 15 rpm, 4 °C before washing twice with PBSB (1 w/v%). Fluorescently in-house labelled HSA (Sigma-Aldrich, St. Louis, MO, USA) with AF-647 (Thermo Scientific, Waltham, MA, USA) was used as secondary reagent to detect binding and incubated with cells for 15 min on ice before washing away non-bound reagent. TfR expression on the cell lines was verified with fluorescently labelled human transferrin (Tf-488, Thermo Scientific, Waltham, MA, USA) and with ab18242 (Abcam, Cambridge, UK), as by manufacturer's instructions. A Gallios™ flow cytometer (Beckman Coulter, Brea, CA, USA) was used to analyze 20,000 events of each sample and data was further analysed in Kaluza (Version 2.1, Beckman Coulter, Brea, CA, USA). All experiments were performed in duplicates and samples normalized to blank cells for comparison between batches.

To analyse pH-dependent binding, SK-OV-3 cells were treated and analyzed as above except for the following differences. $His_6$-[$Z_{TfR}$]-ABD and HSA-647 were preincubated for 20 min and then added to SK-OV-3 cells. After incubation, cells were washed in PBSB (1 w/v%) with different pH in the range 5.0-7.4, respectively, for 30 min at 4 °C. All samples were analysed in triplicate. Experimental set-up with inspiration from Neiveyans et al. [29].

To verify TfR-specific binding, a blocking experiment was performed by co-incubating the $Z_{TfR}$-ABD construct with 5-10× molar excess of $Z_{TfR}$-$His_6$. The SK-OV-3 cells were treated and analyzed as above.

To analyse competitive binding with human transferrin, 25 $\mu$g × mL$^{-1}$ (313 nM) fluorescent labelled transferrin (AF488) (#T13342, Invitrogen, Waltham, MA, USA) was pre-incubated with 100-600 nM of $His_6$-[$Z_{TfR}$]-ABD constructs, respectively, prior to SK-OV-3 cell labelling and flow-cytometric analysis at 488/525 nm and 640/660 nm in triplicate. Additionally, 200 nM of respective $His_6$-[$Z_{TfR}$]-ABD construct was pre-incubated with 15-85 $\mu$g × mL$^{-1}$ (186-1063 nM) of Tf-488 prior to SK-OV-3 cell labelling and flow-cytometric analysis at 488/525 nm and 640/660 nm in triplicate.

Circular dichroism analysis for secondary structure

Circular dichroism (CD) spectroscopy was used to verify the secondary structure content of the affibody molecules in (HE)$_3$-[$Z_{TfR}$]-bio or $His_6$-[$Z_{TfR}$]-ABD format. CD spectra between 195-260 nm at 20 °C were recorded with a Chirascan system (Applied Photophysics, Leatherhead, UK) using a 1 mm High precision cell (110-1P-40 cuvettes, Hellma Analytics, Munich, Germany). Five scans were recorded for each protein sample at a concentration of 0.2 mg × mL$^{-1}$ in PBS.

The thermal melting point for the affibody molecules was determined using a variable temperature measurement (VTM) at 221 nm with a temperature gradient of 5 °C or 1 °C per minute (depend on samples as indicated in results) with five readings at each temperature point. After cooling down to 20 °C, refolding was accessed by recording spectra and comparing to the spectra before denaturation.

Biosensor analysis between $Z_{TfR}$ and TfR of murine and human origin

The affinity and kinetics of the interaction between the $His_6$-[$Z_{TfR}$]-ABD constructs and TfR of human and murine origin was analyzed using surface plasmon resonance (SPR) on a Biacore T200 system (Cytiva, Marlborough, MA, USA) at 25 °C with PBST (0.05% Tween-20) as running buffer. Approximately 1,100 response units (RU) of hTfR-$His_6$ and mTfR-$His_6$ (Sino Biological Inc, Bejing, China), respectively, was immobilized on Series S CM5 chips (Cytiva, Marlborough, MA, USA) via amine coupling as by manufacturer's recommendations. The $Z_{TfR}$ constructs were injected for 300 seconds at 30 $\mu$l × min$^{-1}$ in a dilution series between 582-19.4 nM, in duplicate. Dissociation was monitored for 1,000 seconds before regeneration with 10 mM HCl for 30 seconds, followed by a stabilization period of 60 seconds. Sensorgrams were double-referenced with the blank surface and a buffer injection of PBST and analysed with Biacore evaluation software using 1:1 binding model.

Single amino acid mutagenesis and *E. coli* surface display

The $Z_{TfR\#14}$ and $Z_{TfR\#18}$ affibodies were mutated at 14 positions, respectively, by introducing codons for histidine and the wildtype amino acid of the Z-domain [31], respectively, at each position. Oligos encoding the mutated affibody genes (HT genparts, GenScript, Piscataway, NJ, USA) were subcloned to the *E. coli* display vector pBad2.2 [21] using Gibson assembly according to supplier's recommendations. Four fragments were assembled simultaneously in the Gibson assembly method (New England Biolabs, Ipswich, MA, USA) with a two times molar excess of insert to pBad2.2 vector, and transformed to BL21STAR (DE3) *E. coli* (Thermo Scientific, Waltham, MA, USA) by standard heat shock protocol. Colonies were sequence-verified by sanger sequencing (Eurofins Genomics, Ebersberg, Germany) and further transformed into electrocompetent *E. coli* JK321 [37].

Analysis of TfR-binding using *E. coli* surface display and flow cytometry

Transformed *E. coli* were grown in LB media at 37 °C until OD$_{600}$ reached 0.5 AU before inducing with arabinose (0.6%) and incubation at 25 °C for 16 h protein expression. Cells expressing affibody molecules on the surface were incubated in 50 nM, 100 nM, and 200 nM, respectively, of biotinylated hTfR (#H82E5, Acro Biosystems, Newark, DE, USA) for 45 min. After washing with PBS with 0.01% Pluronic F108 NF surfactant (PBSP), cells were incubated from 15 min on ice, with streptavidin phycoerythrin conjugate (SA-PE; Thermo Scientific, Waltham, MA, USA) and HSA-AF647 (in-house labelled). After washing, cells were analysed on a Gallios™ Flow Cytometer system (Beckman Coulter, Brea, CA, USA) and data analysed in Kaluza (Version 2.1, Beckman Coulter, Brea, CA, USA) for forward/side scatter and relevant fluorophore signals (488/575 nm and 640/660 nm).

Production and labelling of second-generation affibody molecules

Selected clones from the mutagenesis study and a HER2-specific affibody $Z_{HER2:0342}$ [38] as control were subcloned into the pET21a+-vector (Thermo Scientific, Waltham, MA, USA) modified to contain a N-terminal $(HE)_3$-tag and C-terminal cystine using infusion cloning according to supplier's recommendations (Takara, Kusatsu, Japan). The protein constructs were expressed in *E. coli* BL21STAR (DE3) as described above, with the change of carbenicillin (100 $\mu g \times mL^{-1}$). After sonication, the samples were treated at 70 °C for 10 min to precipitate host proteins. After incubation on ice for 20 min, samples were centrifuged for 20 min at 25,000 ×g. Samples were treated with 5 mM tris(2-carboxyethyl)phosphine (TCEP, Sigma-Aldrich/Merck KGaA, Damstadt, Germany) for 30 min and thereafter purified using an ÄKTA start system (Cytiva, Marlborough, MA, USA) with a HisTrap crude column (Cytiva, Marlborough, MA, USA) using running buffer as described above (without imidazole) and elution buffer with 300 mM imidazole in a gradient elution (0-80%). Eluate fractions containing protein were buffer exchanged to PBS (pH 7.0) and concentrated to 2 mg $\times mL^{-1}$ before reducing with TCEP again for 30 min. The reduced proteins were thereafter conjugated to biotin (EZ-Link™ Maleimide-PEG2-Biotin, Thermo Scientific, Waltham, MA, USA) and Fluorescein-5-Maleimide (FITC) (Invitrogen, Waltham, MA, USA), respectively, as by manufacturer's instructions. The secondary structure was verified by CD spectroscopy. Labelling efficacy and concentration after labelling was estimated by SCIEX 4200 MALDI-TOF instrument (SCIEX, Framinghamn, MA, USA) concertation by Pierce™ BCA Protein Assay Kit (Thermo Scientific, Waltham, MA, USA), and degree of labelling was estimated by using a NanoDrop spectrophotometer (NanoDrop Technologies, Wilmington, DE, USA). Purity was analyzed by SDS-PAGE (NuPAGE™ 4 to 12%, Bis-Tris, Invitrogen, Waltham, MA, USA).

Analysis of endocytosis using flow cytometry

Endocytosis was studied using a previously described flow-cytometry method [39]. Briefly, bEnd.3 cells were resuspended in 0.1 mg $\times mL^{-1}$ Dextran-FITC (40 kDa Mw, Merck KGaA, Damstadt, Germany) in ice cold buffers ranging between pH 4.0-5.0 (25 mM Sodium Acetate, 25 mM NaCl, 125 mM KCl, 10 $\mu$M nigericin), pH 5.5-6.5 (25 mM MES, 25 mM NaCl, 125 mM KCl, 10 $\mu$M nigericin), or pH 7.0 (25 mM HEPES, 25 mM NaCl, 125 mM KCl, 10 $\mu$M nigericin), where nigericin was added just before use. Labelled cells were analysed using flow cytometry for FL1 (488/525 nm) and FL2 (488/575 nm) to obtain a standard curve for pH. Each sample was analyzed in triplicate and normalized to blank cells without dextran.

Next, cells were resuspended in ice cold buffer pH 7.4 (25 mM HEPES, 25 mM NaCl, 125 mM KCl) buffer without nigericin and with 250 nM of respective FITC-labelled affibody molecule and incubated for 15 min, 15 rpm, at room temperature before spun down and washed once in buffer of pH 5.0 and once at pH 7.0 (no nigericin) for 5 min at 15 rpm. The sample was resuspended in pH 7.4 buffer and analysed in the flow cytometer with the same settings as for the standard curve.

Transcytosis *in vitro* murine BBB model

A previously described method based on recombinant spider silk nanomembranes with confluent monolayers of brain endothelial cells was used for assessment of TfR-mediated transcytosis [39]. 500 nM of respective FITC-labelled affibody molecule together with a AF647-labelled internal-control IgG2a antibody was added in complete pre-warmed cell media to the silk membrane apical side with (n=3) or without confluent bEnd.3 cell layer (n=3) and placed into a well. Cell media was added to the basal side and incubated for 90 minutes (37 °C, 5% $CO_2$). The apical and basal samples were aspired and together with the starting sample, the fluorescent intensity was measured at 483-14/530-30 nm and 575-20/621-10 nm with 1200/3000 gain at 25 °C in a CLARIOStar Plus (BMG Labtech, Ortenberg, Germany). Values were averaged by technical and biological replicates and signals from complete cell media were subtracted. Statistical analysis was performed in Microsoft excel using two-sided student test with equal variance for the apparent permeability ($p_{app}$, cm $\times$ s$^{-1}$) calculated as by equation 1, where A is the surface area (cm$^2$), dQ/dt the steady-state flux (mmol $\times$ s$^{-1}$), $V_R$ is the volume of the receiver chamber (cm$^3$) and $C_o$ the initial concentration of the affibody (mM).

$$p_{app} = \left(\frac{dQ}{dt}\right) \times \left(\frac{V_R}{A \times C_0}\right) \qquad (1)$$

Further mutagenesis study

A further mutagenesis study of $Z_{TfR\#14}$ and $Z_{TfR\#18}$ was performed to evaluate the binding contribution of amino acids and how substituting to other amino acids affects binding to TfR. Error-prone PCR was used to introduce random mutations in the genes encoding for $Z_{TfR\#14}$ and $Z_{TfR\#18}$, respectively. In addition, a site-directed mutagenesis library was constructed for $Z_{TfR\#14}$, limiting the mutations to the previously 14 randomized positions in helices 1 and 2. Oligos encoding the mutated affibody genes were subcloned to the *E. coli* display vector pBad2.2 [21], and transformed to *E. cloni* EXPRESS BL21(DE3) electrocompetent cells *E. coli* (Lucigen, Wisconsin, USA) by electroporation. Colonies were sequence-verified by sanger sequencing (Eurofins Genomics, Ebersberg, Germany). Transformed *E. coli* were grown in LB media at 37 °C until $OD_{600}$ reached 0.5 AU before inducing with arabinose (0.6%) and incubation at 25 °C for 16 h protein expression. Cells expressing affibody molecules on the surface were incubated in biotinylated hTfR (#H82E5, Acro Biosystems, Newark, DE, USA) for 45 min. After washing with PBS with 0.01% Pluronic F108 NF surfactant (PBSP), cells were incubated on ice, with streptavidin phycoerythrin conjugate (SA-PE; Thermo Scientific, Waltham, MA, USA) and HSA-AF647 (in-house labelled). After washing, binding populations were isolated by FACS using a CytoFLEX SRT cell sorter (Beckman Coulter, Brea, CA, USA). After FACS, individual variants from the sorting output displayed on *E. coli* were analyzed for TfR-binding on a CytoFLEX S flow cytometer (Beckman Coulter, Brea, CA, USA) and data analysed in Kaluza (Version 2.1, Beckman Coulter, Brea, CA, USA). Sanger sequencing was used to identify variants showing improved or retained TfR binding.

## Results

Phage display selections

Affibody molecules were selected against the extracellular domain of recombinant human and murine transferrin receptor from an M13 filamentous phage library containing $3 \times 10^{10}$ affibody molecules. The library was designed to include 14 surface-located randomized positions, distributed over helices 1 and 2 (figure 1A). Five rounds of panning of the library were performed in different tracks against human TfR, murine TfR, or a cross-selection strategy with alternating human and murine TfR, respectively (figure 7). Target concentrations were gradually decreased from 100 to 12.5 nM (or 40 nM in cross-selection tracks) in the final rounds (figure 7). Candidate binders were analysed by phage ELISA for binding to the receptors and 174 colonies from the last or second last cycle of panning were identified by DNA sequencing.

Production of His$_6$-tagged and ABD-fused affibody molecules

Nineteen different affibody molecules from the selections were produced in both a His$_6$-$Z_{TfR}$-ABD and a $Z_{TfR}$-His$_6$ format. The production indicated a higher yield from the smaller format compared to the ABD-fusions. Molecular weight was verified by MS and correlated well with expected values. The purity was verified by SDS-PAGE and all constructs showed the expected alpha helical secondary structure content as measured using circular dichroism (CD) spectroscopy.

Flow cytometry analysis of $Z_{TfR}$ candidates to murine and human cells

The affibody candidates in a $Z_{TfR}$-ABD format were analyzed using flow cytometry for binding to human SK-OV-3 and murine bEnd.3 cell lines expressing TfR (figure 2). Two of the affibody molecules ($Z_{TfR\#14}$ and $Z_{TfR\#18}$) demonstrated binding to both human SK-OV-3 and murine bEnd.3 (figure 2). The two binders originated from the selection track with alternating mTfR and hTfR as target (figure 7). $Z_{TfR\#14}$ and $Z_{TfR\#18}$ showed low sequence homology which is typically an indication of non-overlapping epitopes (figure 1B).

Next, TfR-positive human SK-OV-3 and brain endothelial hCMEC/D3 cells were incubated with a dilution series of $Z_{TfR\#14}$ and $Z_{TfR\#18}$ and binding was analyzed by flow cytometry, showing concentration-dependent signal (figures 8 and 9). It was noted that the signal decreased when cell labelling and analysis was performed at room temperature (data not shown), indicating internalization of the binders prior to addition of secondary detection reagents.

Cell-specific binding was investigated by co-incubating the His$_6$-$Z_{TfR}$-ABD construct with $Z_{TfR}$-His$_6$ at different molar excess ratios, showing substantial decrease in signal in comparison to no blocking (table 1). Moreover, flow cytometry was also used for epitope binning by co-incubating the His$_6$-$Z_{TfR\#14}$-ABD construct with $Z_{TfR\#18}$-His$_6$ and vice versa, showing negligible decrease in binding and thus confirming non-overlapping epitopes (table 1).

Table 1. Flow cytometry data from self-block between $His_6$-$Z_{TfR}$-ABD and $Z_{TfR}$-$His_6$ constructs. Background signal from HSA is included. A shift is seen when 1:5 molar excess of the non-fluorescent clonality is added. In parenthesis the percentage of the signal blocked is shown. The constructs appear not to share epitope where at five times molar excess no change is seen in MFI signal.

| Construct | Blocking 1:0 [MFI] | Blocking 1:1 [MFI] (% loss of signal) | Blocking 1:2 [MFI] (% loss of signal) | Blocking 1:5 [MFI] (% loss of signal) | Block ZTfR#18 1:5 [MFI] |
|---|---|---|---|---|---|
| $Z_{TfR\#14}$-ABD | 15.6 | 13.06 (13%) | 12.89 (17%) | 7.16 (44%) | 0% |
| $Z_{TfR\#18}$-ABD | 8.19 | 8.27 (0%) | 6.3 (23%) | 5.55 (32%) | n.a. |

The high concentration of Tf in blood and results from previous studies demonstrating negative effects on reticulocyte count for transferrin-blocking agents in animal models [17] suggest that competitive binding with transferrin (Tf) is probably not optimal. Another epitope-binning experiment was thus performed, where the $Z_{TfR}$ candidates #2, #4, #10, #14, and #18 in $Z_{TfR}$-ABD format, respectively, were pre-incubated with fluorescently labelled transferrin (Tf-488) prior to analysis of cell-binding. For clones #2, #4, and #10, the Tf-488 signal decreased after co-incubation, whereas no shift in signal was observed for co-incubation with $Z_{TfR\#14}$ and ZTfR#18 (figure 10). Furthermore, the signal from $Z_{TfR\#14}$ and $Z_{TfR\#18}$ was maintained at a 5-fold molar excess of transferrin (figure 3). A non-TfR binding control affibody (Ztaq) [40] was included in the experiment, showing no effect on TfR binding (table 2) and supports the indication that the TfR epitope is not shared between the $Z_{TfR\#14}$, $Z_{TfR\#18}$, and Tf.

Table 2. Mean fluorescence intensity (MFI) from flow cytometry on SK-OV-3 cells co-incubated with Tf-488 and $Z_{TfR\#14}$-ABD, $Z_{TfR\#18}$-ABD and $Z_{taq}$-ABD (negative control). MFI 488/525 nm is corresponding to Tf-binding and MFI 640/660 nm is corresponding to $Z_{TfR}$-ABD-binding.

| $Z_{TfR}$ construct | Concentration $Z_{TfR}$ [nM] | MFI 640/660 nm | Concentration Tf-488 $\mu g \times mL^{-1}$] | MFI 488/525 nm |
|---|---|---|---|---|
| n.a. | 0 | 2.037 | 0 | 0.944 |
| n.a. | 0 | 0.775 | 25 | 7.957 |
| $Z_{TfR\#14}$-ABD | 100 | 7.751 | 25 | 7.220 |
| $Z_{TfR\#14}$-ABD | 300 | 20.537 | 25 | 8.346 |
| $Z_{TfR\#14}$-ABD | 600 | 46.302 | 25 | 9.031 |
| $Z_{TfR\#18}$-ABD | 100 | 5.359 | 25 | 7.859 |
| $Z_{TfR\#18}$-ABD | 300 | 16.549 | 25 | 7.331 |
| $Z_{TfR\#18}$-ABD | 600 | 23.092 | 25 | 10.276 |
| Ztaq-ABD | 200 | 1.621 | 25 | 7.512 |
| Ztaq-ABD | 600 | 3.691 | 25 | 11.432 |

pH-dependent binding to TfR expressing cells

During transcytosis across the brain endothelial cells in the BBB, the pH of the endosomes containing TfR is decreasing to around 5.5 [41,42]. Thus, dissociation of the affibodies from cells at different pH was investigated by employing a flow-cytometric assay similar to previously described studies [29] (figure 4A). The analysis was performed at 4 °C to decrease internalization, and fluorescently labelled Tf (Tf-488) was included for comparison (figure 4B). After incubation of binders with SK-OV-3 cells, cells were washed with buffers of different pH in the range of 5.0-7.4, respectively, for 30 min at 4 °C, followed by analysis of cell-binding using flow cytometry. The pH-dependent binding of Tf-TfR was verified, shown as a decrease in signal for pH below 6.0 (figure 4B). Interestingly, $Z_{TfR\#14}$ showed a pH independent profile and $Z_{TfR\#18}$ had opposite dependence compared to transferrin, where the dissociation appeared slower at lower pH (figure 4B). CD spectroscopy was used to verify secondary structure content, thermal stability, and refolding in the pH range (5.5-7.4). The spectra measured at different pH were overlapping, indicating that the structure was similar and independent of pH in the given range (figure 4C-D). The thermal melting point ($T_m$) was estimated to 56 °C for $Z_{TfR\#14}$ and 58 °C for $Z_{TfR\#18}$ and spectra before and after the variable temperature measurements were overlapping, indicating full refolding.

Surface plasmon resonance analysis of binding between $Z_{TfR}$ and TfR

The affinity of the $Z_{TfR\#14}$-ABD and $Z_{TfR\#18}$-ABD to recombinant TfR was estimated by surface plasmon resonance (SPR) using immobilized extracellular domain of human and murine his-tagged TfR, respectively. Both affibody molecules showed a higher affinity for murine TfR with a $K_D$ of 90 nM for $Z_{TfR\#14}$ and 170 nM for $Z_{TfR\#18}$. For human TfR, the $K_D$ was 150 nM for $Z_{TfR\#14}$ and 710 nM for $Z_{TfR\#18}$ (table 3).

Table 3. Association rate constant ($k_a$), dissociation rate constant ($k_d$), equilibrium dissociation constant ($K_D$), calculated RU max, mass transfer constant (tc), and Chi$^2$ value from fitting SPR data to a 1:1 binding equation.

| Ligand: analyte | $k_a$ [M$^{-1}$s$^{-1}$] | $k_d$ [s$^{-1}$] | $K_D$ [M] | RU (max) | Mass transfer constant (tc) | Chi^2 |
|---|---|---|---|---|---|---|
| hTfR:$Z_{TfR\#14}$ | $7.59 \times 10^3$ | $1.11 \times 10^{-3}$ | $1.46 \times 10^{-7}$ | 56 | $1.06 \times 10^8$ | 3.54 |
| mTfR:$Z_{TfR\#14}$ | $1.20 \times 10^4$ | $1.08 \times 10^{-3}$ | $9.00 \times 10^{-8}$ | 44 | $1.18 \times 10^7$ | 3.82 |
| hTfR:$Z_{TfR\#18}$ | $1.17 \times 10^3$ | $8.30 \times 10^{-4}$ | $7.11 \times 10^{-7}$ | 212 | $6.44 \times 10^{16}$ | 2.06 |
| mTfR:$Z_{TfR\#18}$ | $4.43 \times 10^3$ | $7.62 \times 10^{-4}$ | $1.72 \times 10^{-7}$ | 44 | $1.59 \times 10^{10}$ | 2.77 |

Single amino acid mutagenesis of $Z_{TfR\#14}$ and $Z_{TfR\#18}$

Single amino acid mutagenesis was performed on $Z_{TfR\#14}$ and $Z_{TfR\#18}$ to investigate the contribution of individual residues in binding to TfR. The previously 14 randomized positions of respective affibody were mutated to either histidine, or the wildtype amino acid from the original IgG-binding Z domain [31]. The genes for the mutated affibody molecules were subcloned in fusion to a gene encoding an engineered albumin-binding domain (ABD) [36] into an expression vector for Adhesin Involved in Diffuse Adherence (AIDA) 1-mediated display on the surface of *E. coli* [21]. *E. coli* expressing the different mutants on the surface were analyzed by flow cytometry for assessment of TfR-binding and the original binders ($Z_{TfR\#14}$ and $Z_{TfR\#18}$) were included for comparison. In addition to analysis of TfR-binding, cells were also incubated with saturating concentrations of fluorescently labelled HSA to assess surface expression levels.

For both affibody molecules, most mutations had a negative impact on the binding to TfR (figure 11). However, two mutations with improved binding to TfR for respective binder and with introduction of histidine was found, corresponding to A9H and L27H for $Z_{TfR\#14}$, and M14H and I11N for $Z_{TfR\#18}$ (figures 5 and 11), although M14H seemed to have a negative effect on the cell surface expression.

Analysis of second-generation affibody molecules for TfR

The four mutants ($Z_{TfR\#14\_A9H}$, $Z_{TfR\#14\_L27H}$, $Z_{TfR\#18\_I11N}$ and $Z_{TfR\#18\_M14H}$) that showed improved binding in the mutagenesis study were subcloned for expression of soluble affibody molecules in a (HE)$_3$-$Z_{TfR}$-cys format. In addition, two double mutants were included, corresponding to $Z_{TfR\#14\_A9H-L27H}$ and $Z_{TfR\#18\_I11N\_M14H}$, as well as a control affibody with affinity for the HER2 receptor [38] (table 4). After purification, the C-terminal cysteine in the proteins was conjugated with biotin and FITC, respectively, followed by verification of secondary structure content, molecular mass, thermal stability, and binding to TfR-positive cells (figure 12, table 4-5).

Table 4. Yield for (HE)$_3$-$Z_{TfR}$-cys constructs compared to the yield for the original $Z_{TfR\#14}$ or $Z_{TfR\#18}$ constructs in $Z_{TfR}$-His$_6$ format. Mw is verified by Maldi-TOF.

| Clone | Yield [mg × 100 mL$^{-1}$] | Yield compared to original clonality | Mw expected [Da] | Mw observed [Da] |
|---|---|---|---|---|
| (HE)$_3$-$Z_{TfR\#14\_A9H}$-cys | 0.98 | 0.8 | 7,722 | 7,721 |
| (HE)$_3$-$Z_{TfR\#14\_L27H}$-cys | 2.60 | 2.2 | 7,680 | 7,679 |
| (HE)$_3$-$Z_{TfR\#14\_A9H\_L27H}$-cys | 2.02 | 1.7 | 7,746 | 7,746 |
| (HE)$_3$-$Z_{TfR\#18\_I11N}$-cys | 3.45 | 3.8 | 7,711 | 7,710 |
| (HE)$_3$-$Z_{TfR\#18\_M14H}$-cys | 0.95 | 1.0 | 7,716 | 7,715 |
| (HE)$_3$-$Z_{TfR\#18\_I11N\_M14H}$-cys | 1.31 | 1.4 | 7,716 | 7,716 |
| (HE)$_3$-$Z_{Her2}$-cys | 10.83 | n.a. | 7,739 | 7,738 |

Table 5. Degree of biotinylation, thermal melting point ($T_m$) and refolding for biotinylated single amino acid mutants, $Z_{TfR\#14\_A9H}$, $Z_{TfR\#14\_L27H}$, $Z_{TfR\#14\_A9H\_L27H}$, $Z_{TfR\#18\_I11N}$, $Z_{TfR\#18\_M14H}$, $Z_{TfR\#18l\_11N\_M14H}$, and $Z_{HER2}$.

| Clone | Percentage biotinylated | $T_m$ [°C] | Refolding capability |
|---|---|---|---|
| $(HE)_3$-$Z_{TfR\#14\_A9H}$-biotin | >80% | 64 | Yes |
| $(HE)_3$-$Z_{TfR\#14\_L27H}$-biotin | 100 % | 66 | Partly |
| $(HE)_3$-$Z_{TfR\#14\_A9H\_L27H}$-biotin | >80 % | 62 | Partly |
| $(HE)_3$-$Z_{TfR\#18\_I11N}$-biotin | >60 % | 52 | Partly |
| $(HE)_3$-$Z_{TfR\#18\_M14H}$-biotin | >60 % | 62 | Yes |
| $(HE)_3$-$Z_{TfR\#18\_I11N\_M14H}$-biotin | >50 % | 63 | Yes |
| $(HE)_3$-$Z_{Her2}$-biotin | >70 % | 62 | Yes |

The secondary structure content of the six mutants was similar to $Z_{TfR\#14}$ and $Z_{TfR\#18}$ and thermal stability was either improved or similar (table 5). However, $Z_{TfR\#14\_L27H}$ and $Z_{TfR\#18\_I11N}$ showed non-complete refolding after heat-induced denaturation. All six mutants demonstrated binding to both SK-OV-3 and brain endothelial bEnd.3 cells and the highest signals were observed for $Z_{TfR\#14\_L27H}$ and $Z_{TfR\#18\_M14H}$ (figure 12). The pH-dependency of cell binding was similar for the mutants compared to $Z_{TfR\#14}$ and $Z_{TfR\#18}$, respectively (figure 13).

Flow cytometric endocytosis assay

To study potential endocytosis of the affibody constructs, a previously described flow cytometry-based assay was used [39]. In the assay, the pH sensitivity of FITC is exploited. One emission maximum (FL1 525 nm) of FITC is pH-dependent, whereas the other maximum (FL2 575 nm) is not. Dextran-FITC was added to bEnd.3 cells and incubated at different pH, followed by flow-cytometric analysis of the fluorescence in FL1 and FL2, respectively. The pH was plotted against the FL1/FL2 ratio for a standard curve (figure 14). The FITC-labelled affibody molecules were thereafter incubated with bEnd.3 cells, followed by washing with acidic buffer (pH 5.0) and neutral (pH 7.0) to release remaining membrane-bound affibodies and the FL1 and FL2. After neutralization with buffer, fluorescence was analyzed using flow cytometry.

The TfR-specific BBB shuttle antibody 8D3 has previously been analyzed using the assay, revealing a pH of around 6, which corresponds to recycling TfR endosomes [39,41,42]. The obtained FL1/FL2 ratios for the affibodies corresponded to a pH of around 5-6 for $Z_{TfR\#18}$-derived variants and to a pH of around 6-7 for the $Z_{TfR\#14}$-derived variants (table 6).

Table 6. Estimated pH environment for $Z_{TfR}$-FITC constructs incubated with bEnd.3 cells

| Sample | Calculated pH environment by standard curve | Endocytosis classification |
|---|---|---|
| $(HE)_3$-$Z_{HER2}$-FITC | $5.75 \pm 0.02$ | Late/lysosomal |
| $(HE)_3$-$Z_{TfR\#14\_A9H}$-FITC | $7.15 \pm 0.31$ | Partial cell surface or Vesicle |
| $(HE)_3$-$Z_{TfR\#14\_L27H}$-FITC | $6.94 \pm 0.02$ | Early |
| $(HE)_3$-$Z_{TfR\#14\_A9H\_L27H}$-FITC | $6.18 \pm 0.04$ | Recycling |
| $(HE)_3$-$Z_{TfR\#18\_I11N}$-FITC | $5.13 \pm 0.02$ | Lysosome |
| $(HE)_3$-$Z_{TfR\#18\_M14H}$-FITC | $6.56 \pm 0.02$ | Early |
| $(HE)_3$-$Z_{TfR\#18\_I11N\_M14H}$-FITC | $5.16 \pm 0.01$ | Lysosome |

Transcytosis across a murine *in vitro* BBB model

To study potential transcytosis across the BBB, a previously described in *vitro* assay was employed [39]. The assay is based on transwells with recombinant spider silk nanomembranes supporting confluent monolayers of brain endothelial cells. The FITC-labelled affibody molecules were added to the apical side of bEnd.3 cell membranes together with a fluorescently labelled antibody (IgG-AF647) that is used as an internal control for the barrier integrity of the membranes. The HER2-specific affibody ($Z_{HER2}$) was included as control. After 90 minutes, the media on both sides were collected and the AF647 and FITC fluorescence were measured using a fluorescence spectrophotometer. The fluorescence was finally used to calculate the apparent permeability ($p_{app}$) [39].

All six TfR-specific affibody molecules except for $Z_{TfR\#14\_A9H}$ showed higher apparent permeability than the internal control antibody (table 7, figure 6). Three of the variants also showed higher permeability compared to $Z_{HER2}$, indicating transcytosis over TfR-positive bEnd.3 cells.

Table 7. Transcytosis assay with the $Z_{TfR}$-FITC labelled proteins over bEnd.3 cell membrane on recombinant silk membrane. All samples were analyzed in triplicate. The two-sided student t-test is used for comparing permeability between affibody and the internal control (IgG2a) as membranes might differ in tightness.

| Sample | $p_{app}$ for Z | $p_{app}$ for negative control | Recovery [%] | p-value |
|---|---|---|---|---|
| (HE)$_3$-$Z_{TfR\#14\_A9H}$ -FITC | $5.23 \times 10^{-6} \pm 0.88 \times 10^{-6}$ | $6.17 \times 10^{-6} \pm 1.59 \times 10^{-6}$ | 95 | 0.657 |
| (HE)$_3$-$Z_{TfR\#14\_L27H}$ -FITC | $1.07 \times 10^{-5} \pm 0.23 \times 10^{-5}$ | $4.44 \times 10^{-6} \pm 2.55 \times 10^{-6}$ | 113 | 0.007 |
| (HE)$_3$-$Z_{TfR\#14\_A9H\_L27H}$ -FITC | $5.08 \times 10^{-6} \pm 0.40 \times 10^{-6}$ | $1.01 \times 10^{-6} \pm 2.26 \times 10^{-6}$ | 102 | 0.0000001 |
| (HE)$_3$-$Z_{TfR\#18\_I11N}$ -FITC | $1.11 \times 10^{-5} \pm 0.23 \times 10^{-5}$ | $4.68 \times 10^{-6} \pm 2.34 \times 10^{-6}$ | 117 | 0.050 |
| (HE)$_3$-$Z_{TfR\#18\_M14H}$ -FITC | $5.30 \times 10^{-6} \pm 1.07 \times 10^{-6}$ | $2.92 \times 10^{-6} \pm 0.85 \times 10^{-6}$ | 95 | 0.023 |
| (HE)$_3$-$Z_{TfR\#i8\_I11N\_M14H}$ -FITC | $1.04 \times 10^{-5} \pm 0.27 \times 10^{-5}$ | $3.22 \times 10^{-6} \pm 1.22 \times 10^{-6}$ | 86 | 0.006 |
| (HE)$_3$-$Z_{HER2}$-FITC | $4.14 \times 10^{-6} \pm 2.04 \times 10^{-6}$ | $2.36 \times 10^{-6} \pm 1.69 \times 10^{-6}$ | 90 | 0.289 |

Further mutagenesis study

Variants showing improved or retained TfR binding were identified. The results from the error-prone PCR mutagenesis study of $Z_{TfR\#14}$ and $Z_{TfR\#18}$ are summarized in figures 15a and 16, respectively. The results from the site-directed mutagenesis library constructed for $Z_{TfR\#14}$ (in which the mutations were limited to the previously 14 randomized positions in helices 1 and 2) are summarized in figure 15b.

The amino acid distribution for $Z_{TfR\#14}$-based clones with improved or retained binding is summarized in figure 17. A preferred amino acid distribution is shown in figure 18 based on clones with binding over the cut-off line shown in figure 15.

The amino acid distribution for $Z_{TfR\#18}$-based clones with improved or retained binding is summarized in figure 19. A preferred amino acid distribution is shown in figure 20 based on clones with binding over the cut-off line shown in figure 16.

REFERENCES

1. McConnell, HL; Kersch, CN; Woltjer, RL; Neuwelt, EA. The translational significance of the neurovascular unit. Journal of Biological Chemistry. 2017, 292(3), 762-770. doi:10.1074/jbc.R116.760215

2. Barar, J; Rafi, MA; Pourseif, MM; Omidi, Y. Blood-brain barrier transport machineries and targeted therapy of brain diseases. BioImpacts. 2016, 6(4), 225-248. doi:10.15171/bi.2016.30

3. Saunders, NR; Habgood, MD; Mollgard, K; Dziegielewska, KM. The biological significance of brain barrier mechanisms: help or hindrance in drug delivery to the central nervous system? F1000Res. 2016, 5(313), 313. doi:10.12688/f1000research.7378.1

4. Atwal, JK; Chen, Y; Chiu, C; Mortensen, DL; Meilandt, WJ; Liu, Y; Heise, CE; Hoyte, K; Luk, W; Lu, Y; et al. A Therapeutic Antibody Targeting BACE1 Inhibits Amyloid-$\beta$ Production in Vivo. Sci Transl Med. 2011, 3(84), 4ra43. doi:10.1126/scitranslmed.3002254

5. Yadav, DB; Maloney, JA; Wildsmith, KR; Fuji, RN; Meilandt, WJ; Solanoy, H; Lu, Y; Peng, K; Wilson, B; Chan, P; et al. Widespread brain distribution and activity following i.c.v. infusion of anti-$\beta$-secretase (BACE1) in nonhuman primates. Br J Pharmacol. 2017, 174(22), 4173-4185. doi:10.1111/bph.14021

6. Kissel, K; Hamm, S; Schulz, M; Vecchi, A; Garlanda, C; Engelhardt, B. Immunohistochemical localization of the murine transferrin receptor (TfR) on blood-tissue barriers using a novel anti-TfR monoclonal antibody. Histochem Cell Biol. 1998, 110(1), 63-72. doi:10.1007/s004180050266

7. Hultqvist, G; Syvänen, S; Fang, XT; Lannfelt, L; Sehlin, D. Bivalent brain shuttle increases antibody uptake by monovalent binding to the transferrin receptor. Theranostics. 2017, 7(2), 308-318. doi:10.7150/thno.17155

8. Yu, YJ; Zhang, Y; Kenrick, M; Hoyte, K; Luk, W; Lu, Y; Atwal, J; Elliott, JM; Prabhu, S; Watts, RJ; et al. Boosting Brain Uptake of a Therapeutic Antibody by Reducing Its Affinity for a Transcytosis Target. Sci Transl Med. 2011, 3(84), 84ra44. doi:10.1126/scitranslmed.3002230

9. Niewoehner, J; Bohrmann, B; Collin, L; Urich, E; Sade, H; Maier, P; Rueger, P; Stracke, JO; Lau, W; Tissot, AC; et al. Increased Brain Penetration and Potency of a Therapeutic Antibody Using a Monovalent Molecular Shuttle. Neuron. 2014, 81(1), 49-60. doi:10.1016/j.neuron.2013.10.061

10. Weber, F; Bohrmann, B; Niewoehner, J; Fischer, JAA; Rueger, P; Tiefenthaler, G; Moelleken, J; Bujotzek, A; Brady, K; Singer, T; et al. Brain Shuttle Antibody for Alzheimer's Disease with Attenuated Peripheral Effector Function due to an Inverted Binding Mode. Cell Rep. 2018, 22(1), 149-162. doi:10.1016/j.celrep.2017.12.019

11. Wouters, Y; Jaspers, T; Rué, L; Serneels, L; de Strooper, B; Dewilde, M. VHHs as tools for therapeutic protein delivery to the central nervous system. Fluids Barriers CNS. 2022, 19(1), 79. doi:10.1186/s12987-022-00374-4

12. Lessard, E; Rennie, K; Haqqani, A; Ling, B; Whitfield, J; Paradis, A; Araujo, J; Yoganathan, N; Gillard, J; Stanimirovic, D; et al. Pharmacokinetics and Pharmacodynamic Effect of a Blood-Brain Barrier-Crossing Fusion Protein Therapeutic for Alzheimer's Disease in Rat and Dog. Pharm Res. 2022, 39(7), 1497-1507. doi:10.1007/s11095-022-03285-z

13. Kariolis, MS; Wells, RC; Getz, JA; Kwan, W; Mahon, CS; Tong, R; Kim, J; Srivastava, A; Bedard, C; Henne, KR; et al. Brain delivery of therapeutic proteins using an Fc fragment blood-brain barrier transport vehicle in mice and monkeys. Sci Transl Med. 2020, 12(eaay1359), 1359. doi:10.1126/scitranslmed.aay1359

14. Stocki, P; Szary, JM; Jacobsen, CLM; Demydchuk, M; Northall, L; Moos, T; Walsh, FS; Lynn Rutkowski, J. High efficiency blood-brain barrier transport using a VNAR targeting the Transferrin Receptor 1 (TfR1). bioRxiv. Published online October 28, 2019. doi:10.1101/816900

15. Crook, ZR; Girard, E; Sevilla, GP; Merrill, M; Friend, D; Rupert, PB; Pakiam, F; Nguyen, E; Yin, C; Ruff, RO; et al. A TfR-Binding Cystine-Dense Peptide Promotes Blood-Brain Barrier Penetration of Bioactive Molecules. J Mol Biol. 2020, 432(14), 3989-4009. doi:10.1016/j.jmb.2020.04.002

16. Bien-Ly, N; Yu, YJ; Bumbaca, D; Elstrott, J; Boswell, CA; Zhang, Y; Luk, W; Lu, Y; Dennis, MS; Weimer, RM; et al. Transferrin receptor (TfR) trafficking determines brain uptake of TfR antibody affinity variants. Journal of Experimental Medicine. 2014, 211(2), 233-244. doi:10.1084/jem.20131660

17. Couch, JA; Yu, YJ; Zhang, Y; Tarrant, JM; Fuji, RN; Meilandt, WJ; Solanoy, H; Tong, RK; Hoyte, K; Luk, W; et al. Addressing Safety Liabilities of TfR Bispecific Antibodies That Cross the Blood-Brain Barrier. Sci Transl Med. 2013, 5(183), 183ra57. doi:10.1126/scitranslmed.3005338

18. Faresjö, R; Bonvicini, G; Fang, XT; Aguilar, X; Sehlin, D; Syvänen, S. Brain pharmacokinetics of two BBB penetrating bispecific antibodies of different size. Fluids Barriers CNS. 2021, 18(1), 26. doi:10.1186/s12987-021-00257-0

19. Stahl, S; Gräslund, T; Eriksson Karlström, A; Frejd, FY; Nygren, PÅ; Löfblom, J. Affibody Molecules in Biotechnological and Medical Applications. Trends Biotechnol. 2017, 35(8), 691-712. doi:10.1016/j.tibtech.2017.04.007

20. Engfeldt, T; Renberg, B; Brumer, H; Nygren, PA; Karlström, AE. Chemical synthesis of triple-labelled three-helix bundle binding proteins for specific fluorescent detection of unlabelled protein. ChemBioChem. 2005, 6(6), 1043-1050. doi:10.1002/cbic.200400388

21. Andersson, KG; Persson, J; Stahl, S; Löfblom, J. Autotransporter-Mediated Display of a Naive Affibody Library on the Outer Membrane of Escherichia coli. Biotechnol J. 2019, 14(4), e1800359. doi:10.1002/biot.201800359

22. Grönwall, C; Jonsson, A; Lindström, S; Gunneriusson, E; Stahl, S; Herne, N. Selection and characterization of Affibody ligands binding to Alzheimer amyloid β peptides. JBiotechnol. 2007, 128(1), 162-183. doi:10.1016/j.jbiotec.2006.09.013

23. Affibody AB. Izokibep, a bispecific molecule targeting interleukin-17A (IL-17). Published 2022. Available online: https://www.affibody.se/. (accessed 25 1 2023)

24. Hjelm, LC; Lindberg, H; Stahl, S; Löfblom, J. Construction and Validation of a New Naive Sequestrin Library for Directed Evolution of Binders against Aggregation-Prone Peptides. Int J Mol Sci. 2023, 24(1:836), 836. doi:10.3390/ijms24010836

25. Boutajangout, A; Lindberg, H; Awwad, A; Paul, A; Baitalmal, R; Almokyad, I; Höidén-Guthenberg, I; Gunneriusson, E; Frejd, FY; Härd, T; et al. Affibody-Mediated Sequestration of Amyloid β Demonstrates Preventive Efficacy in a Transgenic Alzheimer's Disease Mouse Model. Front Aging Neurosci. 2019, 11. doi:10.3389/fnagi.2019.00064

26. Meister, SW; Hjelm, LC; Dannemeyer, M; Tegel, H; Lindberg, H; Stahl, S; Löfblom, J. An affibody molecule is actively transported into the cerebrospinal fluid via binding to the transferrin receptor. Int J Mol Sci. 2020, 21(8), 2999. doi:10.3390/ijms21082999

27. Sade, H; Baumgartner, C; Hugenmatter, A; Moessner, E; Freskgard, PO; Niewoehner, J. A human blood-brain barrier transcytosis assay reveals antibody transcytosis influenced by pH-dependent receptor binding. PLoS One. 2014, 9(4). doi:10.1371/journal.pone.0096340

28. Yu, YJ; Atwal, JK; Zhang, Y; Tong, RK; Wildsmith, KR; Tan, C; Bien-Ly, N; Hersom, M; Maloney, JA; Meilandt, WJ; et al. Therapeutic bispecific antibodies cross the blood-brain barrier in nonhuman primates. Sci Transl Med. 2014, 6(261), 261ra154. doi:10.1126/scitranslmed.3009835

29. Neiveyans, M; Melhem, R; Arnoult, C; Bourquard, T; Jarlier, M; Busson, M; Laroche, A; Cerutti, M; Pugnière, M; Ternant, D; et al. A recycling anti transferrin receptor 1 monoclonal antibody as an efficient therapy for erythroleukemia through target up regulation and antibody. MAbs. 2019, 11(3), 359-605. doi:10.1080/19420862.2018.1564510

30. Stocki, P; Szary, J; Rasmussen, CLM; Demydchuk, M; Northall, L; Logan, DB; Gauhar, A; Thei, L; Moos, T; Walsh, FS; et al. Blood-brain barrier transport using a high affinity, brain-selective VNAR antibody targeting transferrin receptor 1. The FASEB Journal. 2021, 35(2), e21172. doi:10.1096/fj.202001787R

31. Nilsson, B; Moks, T; Jansson, B; Abrahmsén, L; Elmblad, A; Holmgren, E; Henrichson, C; Jones, TA; Uhlén, M. A synthetic IgG-binding domain based on staphylococcal protein A. "Protein Engineering, Design and Selection." 1987, 1(2), 107-113. doi:10.1093/protein/1.2.107

32. Ge, YJ; Ou, YN; Deng, YT; Wu, BS; Yang, L; Zhang, YR; Chen, SD; Huang, YY; Dong, Q; Tan, L; et al. Prioritization of Drug Targets for Neurodegenerative Diseases by Integrating Genetic and Proteomic Data From Brain and Blood. Biol Psychiatry. Published online November 2022. doi:10.1016/j.biopsych.2022.11.0022

33. Sokolov, A v.; Dostdar, SA; Attwood, MM; Krasilnikova, AA; Ilina, AA; Nabieva, ASh; Lisitsyna, AA; Chubarev, VN; Tarasov, V v.; Schiöth, HB. Brain Cancer Drug Discovery: Clinical Trials, Drug Classes, Targets, and Combinatorial Therapies. Gottesman M, ed. Pharmacol Rev. 2021, 73(4), 1172-1203. doi:10.1124/pharmrev.121.000317

34. Kontermann, RE. Strategies to Extend Plasma Half-Lives of Recombinant Antibodies. BioDrugs. 2009, 23(2), 93-109. doi:10.2165/00063030-200923020-00003

35. Yu, F; Gudmundsdotter, L; Akal, A; Gunneriusson, E; Frejd, F; Nygren, PA. An affibody-adalimumab hybrid blocks combined IL-6 and tnf-triggered serum amyloid a secretion in vivo. MAbs. 2014, 6(6), 1598-1607. doi:10.4161/mabs.36089

36. Jonsson, A; Dogan, J; Herne, N; Abrahmsén, L; Nygren, PÅ. Engineering of a femtomolar affinity binding protein to human serum albumin. Protein Engineering, Design and Selection. 2008, 21(8), 515-527. doi:10.1093/protein/gzn028

37. Maurer, J; Jose, J; Meyer, TF; Molekulare Biologie, A. Autodisplay: One-Component System for Efficient Surface Display and Release of Soluble Recombinant Proteins from Escherichia coli. JBacteriol. 1997, 179(3), 794-804. doi:10.1128/jb.179.3.794-804-1997

38. Orlova, A; Magnusson, M; Eriksson, TLJ; Nilsson, M; Larsson, B; Höiden-Guthenberg, I; Widström, C; Carlsson, J; Tolmachev, V; Stahl, S; et al. Tumor imaging using a picomolar affinity HER2 binding Affibody molecule. Cancer Res. 2006, 66(8), 4339-4348. doi:10.1158/0008-5472.CAN-05-3521

39. Hjelm, LC; Hedhammar, M; Löfblom, J. In vitro Blood-Brain Barrier Model based on Recombinant Spider Silk-Protein Nanomembranes for Evaluation of Transcytosis capability of biomolecules. Unpublished.

40. Eklund, M; Axelsson, L; Uhlén, M; Nygren, PÅ. Anti-idiotypic protein domains selected from protein A-based affibody libraries. Proteins: Structure, Function and Genetics. 2002, 48(3), 454-462. doi:10.1002/prot.10169

41. Mayle, KM; Le, AM; Kamei, DT. The intracellular trafficking pathway of transferrin. Biochimica et Biophysica Acta (BBA) - General Subjects. 2012, 1820(3), 264-281. doi:10.1016/j.bbagen.2011.09.009

42. Lamb, JE; Ray, F; Ward, JH; Kushner, JP; Kaplan, J. Internalization and subcellular localization of transferrin and transferrin receptors in HeLa cells. J Biol Chem. 1983, 258(14), 8751-8758. Available online: http://www.ncbi.nlm.nih.gov/pubmed/6305999

## Claims

1. A transferrin receptor 1-binding polypeptide comprising the amino acid sequence

$X_9X_{10}X_{11}X_{12}X_{13}X_{14}X_{15}X_{16}X_{17}X_{18}X_{19}X_{20}X_{21}X_{22}NX_{24}X_{25}X_{26}X_{27}X_{28}X_{29}X_{30}X_{31}X_{32}X_{33}X_{34}X_{35}$ (SEQ ID NO:1) wherein

$X_9$ is A, Q, S, H or E,
$X_{10}$ is G, D, E, N or Q,
$X_{11}$ is R, Q, G, H or K,
$X_{12}$ is A, V or T,
$X_{13}$ is K, N or R,
$X_{14}$ is F, L, S, H, I or Y,
$X_{15}$ is E, D or V,
$X_{16}$ is I, N or T,
$X_{17}$ is Y, L or F,
$X_{18}$ is Q, N, E, T, K or D,
$X_{19}$ is L, P or Q,
$X_{20}$ is P, S, Q or T,
$X_{21}$ is N, K, SorY,
$X_{22}$ is L, M or Q,
$X_{24}$ is M, N, R, K, Q or H,
$X_{25}$ is F, I, L, Y, R, K, T, S, Q or N,
$X_{26}$ is Q, P or H,
$X_{27}$ is L, H, V, Q, R or K,
$X_{28}$ is F, V, Yor H,
$X_{29}$ is A or T,
$X_{30}$ is F, I or Y,
$X_{31}$ is H, R, Q, Y, L, D or N,
$X_{32}$ is H, Y, P, N, L, R, E or K,
$X_{33}$ is S, G, T or I,
$X_{34}$ is L, S, F or V and
$X_{35}$ is F, L, K, Y, R, S, D, E, N.

2. The polypeptide of claim 1, further comprising the amino acid sequence $X_4X_5X_6X_7X_8$ at the N terminus of SEQ ID NO:1, wherein

$X_4$ is K, R or E, preferably K,
$X_5$ is F, I, L or S, preferably F or S, more preferably F,
$X_6$ is N or S, preferably N,
$X_7$ is K or R, preferably K, and
$X_8$ is E, V or D, preferably E or V, more preferably E.

3. The polypeptide of claim 2, wherein the N terminus of SEQ ID NO:1 is further extended by the sequence VDN.

4. The polypeptide of any one of the preceding claims, further comprising the amino acid sequence $X_{36}X_{37}X_{38}X_{39}X_{40}$ at the C terminus of SEQ ID NO:1, wherein

$X_{36}$ is D, E or V, preferably D or V,
$X_{37}$ is D or N, preferably D,
$X_{38}$ is P or S, preferably P,
$X_{39}$ is S, T, I or G, preferably S or T, more preferably S, and
$X_{40}$ is Q, L, E or H, preferably Q, E or H, more preferably Q or H.

5. The polypeptide of claim 4, wherein the C terminus of SEQ ID NO:1 is further extended by the sequence $X_{41}X_{42}X_{43}X_{44}X_{45}X_{46}X_{47}X_{48}X_{49}X_{50}X_{51}X_{52}X_{53}X_{54}$, wherein

$X_{41}$ is S, N, R or G, preferably S or N,
$X_{42}$ is A, V or T, preferably A,
$X_{43}$ is N or D, preferably N,
$X_{44}$ is L, M or F, preferably L or M,
$X_{45}$ is L, P or Q, preferably L or P,
$X_{46}$ is A, P, G, T or S, preferably A, P or T,
$X_{47}$ is E, D or K, preferably E or K,
$X_{48}$ is A, V or T, preferably A,
$X_{49}$ is K, R, N or I, preferably K or R,
$X_{50}$ is K, N, E or M, preferably K or M,
$X_{51}$ is L, I, Q or P, preferably L,
$X_{52}$ is N, I, D or K, preferably N,
$X_{53}$ is D, N or E, preferably D or N, and
$X_{54}$ is A, T, P or V, preferably A.

6. The polypeptide of claim 5, wherein the C terminus of SEQ ID NO:1 is still further extended by the sequence $QAX_{57}K$, wherein $X_{57}$ is P or S, preferably P.

7. The polypeptide of any one of the preceding claims, wherein

$X_9$ is A, Q, S, H or E,
$X_{11}$ is R, H or K,
$X_{12}$ is A, V or T,
$X_{13}$ is K or R,
$X_{14}$ is F, H, I or Y,
$X_{15}$ is E or V,
$X_{16}$ is I,
$X_{17}$ is Y, L or F,
$X_{18}$ is Q, N, E, T, K or D,
$X_{19}$ is L or P,
$X_{20}$ is P, S or T,
$X_{21}$ is N,
$X_{22}$ is L, M or Q,
$X_{25}$ is F, I, L, R, K, T, S, Q or N,
$X_{26}$ is Q,

$X_{27}$ is L, H, Q, R or K,
$X_{30}$ is F or Y,
$X_{31}$ is H, Q, D or N,
$X_{33}$ is S or G,
$X_{34}$ is L or S and/or
$X_{35}$ is F, L, K, Y, S, D, E or N.

8. The polypeptide of any one of the preceding claims, wherein

$X_9$ is A, Q or H,
$X_{10}$ is G, D or E,
$X_{11}$ is R or H,
$X_{12}$ is A or T,
$X_{13}$ is K or R,
$X_{14}$ is F or H,
$X_{15}$ is E or V,
$X_{16}$ is I,
$X_{17}$ is Y or F,
$X_{18}$ is Q, N or E,
$X_{19}$ is L or P,
$X_{20}$ is P or T,
$X_{21}$ is N,
$X_{24}$ is M,
$X_{25}$ is F, I, R or K, preferably F, R or K
$X_{26}$ is Q,
$X_{27}$ is L, R, H or K,
$X_{28}$ is F,
$X_{30}$ is F or Y,
$X_{31}$ is H, Q or D,
$X_{32}$ is H, R or K,
$X_{33}$ is S,
$X_{34}$ is L or S and/or
$X_{35}$ is F, D or E.

9. A fusion protein or conjugate comprising:

- a first part comprising a polypeptide according to any one of claims 1-15; and
- a second part having a therapeutic activity.

10. The fusion protein or conjugate of claim 9, wherein the therapeutic activity is based on binding to a target selected from the group consisting of amyloid β, Tau, apolipoprotein E (ApoE), beta-secretase 1 (BACE1), gamma-secretase, complement factor C1s, presenilin 1, presenilin 2, nicastrin, alpha-synuclein, sortilin, progranulin, prosaposin and Bri.

11. The polypeptide, fusion protein or conjugate of any one of the preceding claims for use as a medicament.

12. A pharmaceutical composition comprising the polypeptide, fusion protein or conjugate of any one of the preceding claims.

13. The polypeptide, fusion protein, conjugate or pharmaceutical composition of any one of the preceding claims for use in a method of treatment of a neurological disorder, such as Parkinson's disease or dementia.

14. The polypeptide, fusion protein, conjugate or pharmaceutical composition for use according to claim 13, wherein said dementia is Lewy body dementia or Alzheimer's disease.

15. The polypeptide, fusion protein, conjugate or pharmaceutical composition of any one of claims 1-12 for use in a method of treatment of a brain cancer.

A

B

| | 1 | | 10 | | 20 | | 30 | | 40 | | 50 | 58 | |
Z library  VDNKFNKEXXXAXXEIXXLPNLNXXQXXAFXXSLXDDPSQSANLLAEAKKLNDAQAPK  (SEQ ID NO:6)

ZTfR#14  · · · · · · · AGR·KF· ·YQ· · · ·MF·LF· ·HH· ·F· · · · · · · · · · · · · · · · · · · · · ·  (SEQ ID NO:7)

ZTfR#18  · · · · · · · RHI·FM· ·HF· · · · ·ID·NY· ·DF· ·K· · · · · · · · · · · · · · · · · · · · · ·  (SEQ ID NO:8)

Fig. 1

| C | Average mean bEnd.3 | Average mean SK-OV-3 |
|---|---|---|
| ■Blank cells | 1.00 | 1.00 |
| ■HSA-647 | 1.72 | 1.72 |
| ■Ztaq-ABD | 2.20 | 2.16 |
| ■scFv8D3-Z_serum-ABD | 4.13 | 2.27 |
| ■Z_TTR#1-ABD | 2.40 | 1.93 |
| ■Z_TTR#2-ABD | 2.09 | 3.09 |
| ■Z_TTR#3-ABD | 2.65 | 2.26 |
| ■Z_TTR#4-ABD | 1.47 | 4.20 |
| ■Z_TTR#6-ABD | 1.49 | 2.03 |
| ■Z_TTR#7-ABD | 1.80 | 2.23 |
| ■Z_TTR#8-ABD | 2.05 | 1.86 |
| ■Z_TTR#9-ABD | 1.72 | 1.57 |
| ■Z_TTR#10-ABD | 1.69 | 4.33 |
| ■Z_TTR#11-ABD | 2.02 | 2.93 |
| ■Z_TTR#12-ABD | 1.79 | 2.69 |
| ■Z_TTR#13-ABD | 2.02 | 2.85 |
| ■Z_TTR#14-ABD | 6.58 | 47.52 |
| ■Z_TTR#16-ABD | 2.32 | 2.15 |
| ■Z_TTR#17-ABD | 2.33 | 2.06 |
| ■Z_TTR#18-ABD | 7.20 | 28.15 |
| ■Z_TTR#19-ABD | 1.70 | 1.96 |

Fig. 2

Fig. 3

Fig. 4

Fig. 5

Fig. 6

Fig. 7

Fig. 8

Fig. 9

Fig. 10

Fig. 11

Fig. 12

Fig. 13

y = 0.1861x + 0.8289

$R^2 = 0.9653$

Fig. 14

Single clones from ZTfR#14_R4A_ePCR_library (1/4)

Single clones from ZTfR#14_R4A_ePCR_library (2/4)

Single clones from ZTfR#14_R4B_ePCR_library (3/4)

Single clones from ZTfR#14_R4B_ePCR_library (4/4)

Fig. 15a

Fig. 15b

Fig. 16

| Position | Original AA | Improved AA | Position | Original AA | Improved AA |
|---|---|---|---|---|---|
| 1 | V | V | 33 | S | S, G, T, I |
| 2 | D | D | 34 | L | L, S, F, V, |
| 3 | N | N | **35** | F | F, L, K, Y, R, S, D, E, N |
| 4 | K | K, R, E | 36 | D | D, E, V |
| 5 | F | F, I, L, S | 37 | D | D, N |
| 6 | N | N, S | 38 | P | P, S |
| 7 | K | K, R | 39 | S | S, T, I, G |
| 8 | E | E, V, D | 40 | Q | Q, L, E, H |
| **9** | A | A, Q, S, H, E | 41 | S | S, N, R, G |
| **10** | G | G, D, E, N, Q | 42 | A | A, V, T |
| **11** | R | R, Q, G, H, K | 43 | N | N, D |
| 12 | A | A, V, T | 44 | L | L, M, F |
| **13** | K | K, N, R | 45 | L | L, P, Q |
| 14 | F | F, L, S, H, I, Y | 46 | A | A, P, G, T, S |
| 15 | E | E, D, V | 47 | E | E, D, K |
| 16 | I | I, N, T | 48 | A | A, V, T |
| **17** | Y | Y, L, F | 49 | K | K, R, N, I |
| **18** | Q | Q, N, E, T, K, D | 50 | K | K, N, E, M |
| 19 | L | L, P, Q | 51 | L | L, I, Q, P |
| 20 | P | P, S, Q, T | 52 | N | N, I, D, K |
| 21 | N | N, K, S, Y | 53 | D | D, N, E |
| 22 | L | L, M, Q | 54 | A | A, T, P, V |
| 23 | N | N | 55 | Q | Q |
| **24** | M | M, N, R, K, Q, H | 56 | A | A |
| **25** | F | F, I, L, Y, R, K, T, S, Q, N | 57 | P | P, S |
| 26 | Q | Q, P, H | 58 | K | K |
| **27** | L | L, H, V, Q, R, K | | | |
| **28** | F | F, V, Y, H | | | |
| 29 | A | A, T | | | |
| 30 | F | F, I, Y | | | |
| **31** | H | H, R, Q, Y, L, D, N | | | |
| **32** | H | H, Y, P, N, L, R, E, K | | | |

# Fig. 17

| Position | Original AA | Improved AA | | Position | Original AA | Improved AA |
|---|---|---|---|---|---|---|
| 1 | V | V | | 33 | S | S |
| 2 | D | D | | 34 | L | S, L |
| 3 | N | N | | 35 | F | F, D, E |
| 4 | K | K | | 36 | D | D, V |
| 5 | F | F | | 37 | D | D |
| 6 | N | N | | 38 | P | P |
| 7 | K | K | | 39 | S | S |
| 8 | E | E | | 40 | Q | Q, H |
| 9 | A | A, Q, H | | 41 | S | S, N |
| 10 | G | G, D, E | | 42 | A | A |
| 11 | R | R, H | | 43 | N | N |
| 12 | A | A, T | | 44 | L | L, M |
| 13 | K | K, R | | 45 | L | L, P |
| 14 | F | F, H | | 46 | A | A, P, T |
| 15 | E | E, V | | 47 | E | E, K |
| 16 | I | I | | 48 | A | A |
| 17 | Y | Y, F | | 49 | K | K, R |
| 18 | Q | Q, N, E | | 50 | K | K, M |
| 19 | L | L, P | | 51 | L | L |
| 20 | P | P, T | | 52 | N | N |
| 21 | N | N | | 53 | D | D, N |
| 22 | L | L, M, Q | | 54 | A | A |
| 23 | N | N | | 55 | Q | Q |
| 24 | M | M | | 56 | A | A |
| 25 | F | F, R, K | | 57 | P | P |
| 26 | Q | Q | | 58 | K | K |
| 27 | L | L, R, K | | | | |
| 28 | F | F | | | | |
| 29 | A | A, T | | | | |
| 30 | F | F, Y | | | | |
| 31 | H | H, Q, D | | | | |
| 32 | H | H, R, K | | | | |

## Fig. 18

| Position | Original AA | Improved AA | | Position | Original AA | Improved AA |
|---|---|---|---|---|---|---|
| 1 | V | V | | 33 | S | S, R |
| 2 | D | D | | 34 | L | L, F |
| 3 | N | N | | 35 | K | K, M, R |
| 4 | K | K, I | | 36 | D | D |
| 5 | F | F | | 37 | D | D, E |
| 6 | N | N | | 38 | P | P, L, T |
| 7 | K | K | | 39 | S | S |
| 8 | E | E | | 40 | Q | Q, L, H |
| 9 | R | R | | 41 | S | S |
| 10 | H | H, L, R, Y | | 42 | A | A |
| 11 | I | N, I | | 43 | N | N, T |
| 12 | A | A, S, E, V | | 44 | L | L |
| 13 | F | F, H | | 45 | L | L |
| 14 | M | M, H | | 46 | A | A |
| 15 | E | E | | 47 | E | E |
| 16 | I | I, T | | 48 | A | A, T |
| 17 | H | H, P, N, R | | 49 | K | K |
| 18 | F | F, H, Y, S | | 50 | K | K, E, M |
| 19 | L | L, Q | | 51 | L | L, P |
| 20 | P | P, S, Q | | 52 | N | N |
| 21 | N | N, Y | | 53 | D | D, Y |
| 22 | L | L, M, Q | | 54 | A | A, T |
| 23 | N | N, S, Y | | 55 | Q | Q |
| 24 | I | I, F, V | | 56 | A | A, V |
| 25 | D | D, V | | 57 | P | P, S |
| 26 | Q | Q, R, H | | 58 | K | K |
| 27 | N | N, I, Y | | | | |
| 28 | Y | Y, N | | | | |
| 29 | A | A, T | | | | |
| 30 | F | F | | | | |
| 31 | D | D, E, G, Y | | | | |
| 32 | F | F, I, L | | | | |

Fig. 19

| Position | Original AA | Improved AA | Position | Original AA | Improved AA |
|---|---|---|---|---|---|
| 1 | V | V | 33 | S | S |
| 2 | D | D | 34 | L | L, F |
| 3 | N | N | **35** | K | K, M |
| 4 | K | K | 36 | D | D |
| 5 | F | F | 37 | D | D |
| 6 | N | N | 38 | P | P, T |
| 7 | K | K | 39 | S | S |
| 8 | E | E | 40 | Q | Q |
| **9** | R | R | 41 | S | S |
| **10** | H | R, H, Y | 42 | A | A |
| **11** | I | I, N | 43 | N | N |
| 12 | A | A, S, V | 44 | L | L |
| **13** | F | F | 45 | L | L |
| **14** | M | M, H | 46 | A | A |
| 15 | E | E | 47 | E | E |
| 16 | I | I | 48 | A | A, T |
| **17** | H | H, N | 49 | K | K |
| **18** | F | F, H | 50 | K | K, M |
| 19 | L | L | 51 | L | L |
| 20 | P | P, Q | 52 | N | N |
| 21 | N | N, Y | 53 | D | D, Y |
| 22 | L | L, I, Q | 54 | A | A |
| 23 | N | N, S | 55 | Q | Q |
| **24** | I | I | 56 | A | A |
| **25** | D | D, V | 57 | P | P |
| 26 | Q | Q, H | 58 | K | K |
| **27** | N | N, I | | | |
| **28** | Y | Y, N | | | |
| 29 | A | A, T | | | |
| 30 | F | F | | | |
| **31** | D | D, Y | | | |
| **32** | F | F, I, L | | | |

Fig. 20

**EUROPEAN SEARCH REPORT**

Europäisches
Patentamt
European
Patent Office
Office européen
des brevets

Application Number

EP 23 15 9544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/281021 A1 (NOVO NORDISK AS [DK]) 12 January 2023 (2023-01-12) * Example 1; page 21, line 25; page 24, lines 6-8; SEQ ID NO: 8 * ----- | 1-15 | INV. C07K14/31 C07K14/705 A61P25/00 A61P25/16 A61P35/00 A61K47/64 |
| A | WO 2022/115715 A1 (HUTCHINSON FRED CANCER RES [US]; BLAZE BIOSCIENCE INC [US] ET AL.) 2 June 2022 (2022-06-02) * [0170], [0297] * ----- | 1-15 | |
| A | WO 2016/131987 A1 (AFFIBODY AB [SE]) 25 August 2016 (2016-08-25) * claims 10-11 and 14; page 31, lines 7-36 * ----- | 1-15 | |
| A | MEISTER SEBASTIAN W. ET AL: "An Affibody Molecule Is Actively Transported into the Cerebrospinal Fluid via Binding to the Transferrin Receptor", INTERNATIONAL JOURNAL OF MOLECULAR SCIENCES, vol. 21, no. 8, 23 April 2020 (2020-04-23) , page 2999, XP093046840, Basel, CH ISSN: 1661-6596, DOI: 10.3390/ijms21082999 * Abstract; Figure 1A; p. 2-12 * ----- | 1-15 | **TECHNICAL FIELDS SEARCHED (IPC)** C07K C12R A61P A61K |
| A | STÅHL STEFAN ET AL: "Affibody Molecules in Biotechnological and Medical Applications", TRENDS IN BIOTECHNOLOGY, ELSEVIER PUBLICATIONS, CAMBRIDGE, GB, vol. 35, no. 8, 14 May 2017 (2017-05-14), pages 691-712, XP085136000, ISSN: 0167-7799, DOI: 10.1016/J.TIBTECH.2017.04.007 * p.691-708 * ----- -/-- | 1-15 | |

The present search report has been drawn up for all claims

| Place of search | Date of completion of the search | Examiner |
|---|---|---|
| Munich | 14 August 2023 | Benzerari, Maëlis |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

1

**page 1 of 2**

Europäisches
Patentamt

European
Patent Office

Office européen
des brevets

# EUROPEAN SEARCH REPORT

Application Number

EP 23 15 9544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | J. LÖFBLOM ET AL: "Affibody molecules: Engineered proteins for therapeutic, diagnostic and biotechnological applications", FEBS LETTERS, vol. 584, no. 12, 1 June 2010 (2010-06-01), pages 2670-2680, XP055054512, ISSN: 0014-5793, DOI: 10.1016/j.febslet.2010.04.014 * Abstract; p. 2671, "2. Affibody technology"; Figure 2 * ----- | 1-15 | |
| A | GRONWALL ET AL: "Selection and characterization of Affibody ligands binding to Alzheimer amyloid @b peptides", JOURNAL OF BIOTECHNOLOGY, ELSEVIER, AMSTERDAM NL, vol. 128, no. 1, 23 December 2006 (2006-12-23), pages 162-183, XP005734691, ISSN: 0168-1656, DOI: 10.1016/J.JBIOTEC.2006.09.013 * Abstract; p. 171 "Selection of Affinity variants"; Figure 1 * ----- | 1-15 | TECHNICAL FIELDS SEARCHED (IPC) |

The present search report has been drawn up for all claims

| Place of search | Date of completion of the search | Examiner |
|---|---|---|
| Munich | 14 August 2023 | Benzerari, Maëlis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 9544

This annex lists the patent family members relating to the patent documents cited in  the above-mentioned European search report.
The members are as contained in the European Patent Office EDP file on
The European Patent Office is in no way liable for these particulars which are merely given for the purpose of information.

14-08-2023

| Patent document cited in search report | | Publication date | Patent family member(s) | | Publication date |
|---|---|---|---|---|---|
| WO 2023281021 | A1 | 12-01-2023 | NONE | | |
| WO 2022115715 | A1 | 02-06-2022 | AU | 2021386254 A1 | 29-06-2023 |
| | | | CA | 3195315 A1 | 02-06-2022 |
| | | | IL | 303152 A | 01-07-2023 |
| | | | WO | 2022115715 A1 | 02-06-2022 |
| WO 2016131987 | A1 | 25-08-2016 | EP | 3258950 A1 | 27-12-2017 |
| | | | JP | 6979577 B2 | 15-12-2021 |
| | | | JP | 2018513833 A | 31-05-2018 |
| | | | US | 2018057551 A1 | 01-03-2018 |
| | | | US | 2020172588 A1 | 04-06-2020 |
| | | | US | 2021355180 A1 | 18-11-2021 |
| | | | WO | 2016131987 A1 | 25-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016131987 A


**Non-patent literature cited in the description**

- **MCCONNELL, HL ; KERSCH, CN ; WOLTJER, RL ; NEUWELT, EA.** The translational significance of the neurovascular unit. *Journal of Biological Chemistry,* 2017, vol. 292 (3), 762-770
- **BARAR, J ; RAFI, MA ; POURSEIF, MM ; OMIDI, Y.** Blood-brain barrier transport machineries and targeted therapy of brain diseases. *BioImpacts,* 2016, vol. 6 (4), 225-248
- **SAUNDERS, NR ; HABGOOD, MD ; MOLLGARD, K ; DZIEGIELEWSKA, KM.** The biological significance of brain barrier mechanisms: help or hindrance in drug delivery to the central nervous system?. *F1000Res,* 2016, vol. 5 (313), 313
- **ATWAL, JK ; CHEN, Y ; CHIU, C ; MORTENSEN, DL ; MEILANDT, WJ ; LIU, Y ; HEISE, CE ; HOYTE, K ; LUK, W ; LU, Y et al.** A Therapeutic Antibody Targeting BACE1 Inhibits Amyloid-β Production in Vivo. *Sci Transl Med.,* 2011, vol. 3 (84), 4-43
- **YADAV, DB ; MALONEY, JA ; WILDSMITH, KR ; FUJI, RN ; MEILANDT, WJ ; SOLANOY, H ; LU, Y ; PENG, K ; WILSON, B ; CHAN, P et al.** Widespread brain distribution and activity following i.c.v. infusion of anti-β-secretase (BACE1) in nonhuman primates. *Br J Pharmacol,* 2017, vol. 174 (22), 4173-4185
- **KISSEL, K ; HAMM, S ; SCHULZ, M ; VECCHI, A ; GARLANDA, C ; ENGELHARDT, B.** Immunohistochemical localization of the murine transferrin receptor (TfR) on blood-tissue barriers using a novel anti-TfR monoclonal antibody. *Histochem Cell Biol.,* 1998, vol. 110 (1), 63-72
- **HULTQVIST, G ; SYVÄNEN, S ; FANG, XT ; LANNFELT, L ; SEHLIN, D.** Bivalent brain shuttle increases antibody uptake by monovalent binding to the transferrin receptor. *Theranostics.,* 2017, vol. 7 (2), 308-318
- **YU, YJ ; ZHANG, Y ; KENRICK, M ; HOYTE, K ; LUK, W ; LU, Y ; ATWAL, J ; ELLIOTT, JM ; PRABHU, S ; WATTS, RJ et al.** Boosting Brain Uptake of a Therapeutic Antibody by Reducing Its Affinity for a Transcytosis Target. *Sci Transl Med.,* 2011, vol. 3 (84), 84-44
- **NIEWOEHNER, J ; BOHRMANN, B ; COLLIN, L ; URICH, E ; SADE, H ; MAIER, P ; RUEGER, P ; STRACKE, JO ; LAU, W ; TISSOT, AC et al.** Increased Brain Penetration and Potency of a Therapeutic Antibody Using a Monovalent Molecular Shuttle. *Neuron,* 2014, vol. 81 (1), 49-60
- **WEBER, F ; BOHRMANN, B ; NIEWOEHNER, J ; FISCHER, JAA ; RUEGER, P ; TIEFENTHALER, G ; MOELLEKEN, J ; BUJOTZEK, A ; BRADY, K ; SINGER, T; et al.** Brain Shuttle Antibody for Alzheimer's Disease with Attenuated Peripheral Effector Function due to an Inverted Binding Mode. *Cell Rep.,* 2018, vol. 22 (1), 149-162
- **WOUTERS, Y ; JASPERS, T ; RUÉ, L ; SERNEELS, L ; DE STROOPER, B ; DEWILDE, M.** VHHs as tools for therapeutic protein delivery to the central nervous system. *Fluids Barriers CNS.,* 2022, vol. 19 (1), 79
- **LESSARD, E ; RENNIE, K ; HAQQANI, A ; LING, B ; WHITFIELD, J ; PARADIS, A ; ARAUJO, J ; YOGANATHAN, N ; GILLARD, J ; STANIMIROVIC, D et al.** Pharmacokinetics and Pharmacodynamic Effect of a Blood-Brain Barrier-Crossing Fusion Protein Therapeutic for Alzheimer's Disease in Rat and Dog. *Pharm Res.,* 2022, vol. 39 (7), 1497-1507
- **KARIOLIS, MS ; WELLS, RC ; GETZ, JA ; KWAN, W ; MAHON, CS ; TONG, R ; KIM, J ; SRIVASTAVA, A ; BEDARD, C ; HENNE, KR et al.** Brain delivery of therapeutic proteins using an Fc fragment blood-brain barrier transport vehicle in mice and monkeys. *Sci Transl Med.,* 2020, vol. 12 (eaay1359), 1359
- **STOCKI, P ; SZARY, JM ; JACOBSEN, CLM ; DEMYDCHUK, M ; NORTHALL, L ; MOOS, T ; WALSH, FS ; LYNN RUTKOWSKI.** J. High efficiency blood-brain barrier transport using a VNAR targeting the Transferrin Receptor 1 (TfR1). *bioRxiv.,* 28 October 2019

- **CROOK, ZR ; GIRARD, E ; SEVILLA, GP ; MER-RILL, M ; FRIEND, D ; RUPERT, PB ; PAKIAM, F ; NGUYEN, E ; YIN, C ; RUFF, RO et al.** A TfR-Binding Cystine-Dense Peptide Promotes Blood-Brain Barrier Penetration of Bioactive Molecules. *J Mol Biol.,* 2020, vol. 432 (14), 3989-4009
- **BIEN-LY, N ; YU, YJ ; BUMBACA, D ; ELSTROTT, J ; BOSWELL, CA ; ZHANG, Y ; LUK, W ; LU, Y ; DENNIS, MS ; WEIMER, RM et al.** Transferrin receptor (TfR) trafficking determines brain uptake of TfR antibody affinity variants. *Journal of Experimental Medicine,* 2014, vol. 211 (2), 233-244
- **COUCH, JA ; YU, YJ ; ZHANG, Y ; TARRANT, JM ; FUJI, RN ; MEILANDT, WJ ; SOLANOY, H ; TONG, RK ; HOYTE, K ; LUK, W et al.** Addressing Safety Liabilities of TfR Bispecific Antibodies That Cross the Blood-Brain Barrier. *Sci Transl Med.,* 2013, vol. 5 (183), 183-57
- **FARESJÖ, R ; BONVICINI, G ; FANG, XT ; AGUI-LAR, X ; SEHLIN, D ; SYVÄNEN, S.** Brain pharmacokinetics of two BBB penetrating bispecific antibodies of different size. *Fluids Barriers CNS.,* 2021, vol. 18 (1), 26
- **STAHL, S ; GRÄSLUND, T ; ERIKSSON KARL-STRÖM, A ; FREJD, FY ; NYGREN, PÅ ; LÖFB-LOM, J.** Affibody Molecules in Biotechnological and Medical Applications. *Trends Biotechnol.,* 2017, vol. 35 (8), 691-712
- **ENGFELDT, T ; RENBERG, B ; BRUMER, H ; NY-GREN, PA ; KARLSTRÖM, AE.** Chemical synthesis of triple-labelled three-helix bundle binding proteins for specific fluorescent detection of unlabelled protein. *ChemBioChem.,* 2005, vol. 6 (6), 1043-1050
- **ANDERSSON, KG ; PERSSON, J ; STAHL, S ; LÖFBLOM, J.** Autotransporter-Mediated Display of a Naive Affibody Library on the Outer Membrane of Escherichia coli. *Biotechnol J.,* 2019, vol. 14 (4), e1800359
- **GRÖNWALL, C ; JONSSON, A ; LINDSTRÖM, S ; GUNNERIUSSON, E ; STAHL, S ; HERNE, N.** Selection and characterization of Affibody ligands binding to Alzheimer amyloid β peptides. *JBiotechnol,* 2007, vol. 128 (1), 162-183
- **AFFIBODY AB.** *Izokibep, a bispecific molecule targeting interleukin-17A (IL-17),* 2022, https://www.affibody.se
- **HJELM, LC ; LINDBERG, H ; STAHL, S ; LÖFB-LOM, J.** Construction and Validation of a New Naive Sequestrin Library for Directed Evolution of Binders against Aggregation-Prone Peptides. *Int J Mol Sci,* 2023, vol. 24 (1-836), 836
- **BOUTAJANGOUT, A ; LINDBERG, H ; AWWAD, A ; PAUL, A ; BAITALMAL, R ; ALMOKYAD, I ; HÖIDÉN-GUTHENBERG, I ; GUNNERIUSSON, E ; FREJD, FY ; HÄRD, T et al.** Affibody-Mediated Sequestration of Amyloid β Demonstrates Preventive Efficacy in a Transgenic Alzheimer's Disease Mouse Model. *Front Aging Neurosci.,* 2019, vol. 11
- **MEISTER, SW ; HJELM, LC ; DANNEMEYER, M ; TEGEL, H ; LINDBERG, H ; STAHL, S ; LÖFBLOM, J.** An affibody molecule is actively transported into the cerebrospinal fluid via binding to the transferrin receptor. *Int J Mol Sci.,* 2020, vol. 21 (8), 2999
- **SADE, H ; BAUMGARTNER, C ; HUGENMATTER, A ; MOESSNER, E ; FRESKGARD, PO ; NIE-WOEHNER, J.** A human blood-brain barrier transcytosis assay reveals antibody transcytosis influenced by pH-dependent receptor binding. *PLoS One,* 2014, vol. 9 (4
- **YU, YJ ; ATWAL, JK ; ZHANG, Y ; TONG, RK ; WILDSMITH, KR ; TAN, C ; BIEN-LY, N ; HERSOM, M ; MALONEY, JA ; MEILANDT, WJ et al.** Therapeutic bispecific antibodies cross the blood-brain barrier in nonhuman primates. *Sci Transl Med.,* 2014, vol. 6 (261), 261-154
- **NEIVEYANS, M ; MELHEM, R ; ARNOULT, C ; BOURQUARD, T ; JARLIER, M ; BUSSON, M ; LA-ROCHE, A ; CERUTTI, M ; PUGNIÈRE, M ; TERN-ANT, D et al.** A recycling anti transferrin receptor 1 monoclonal antibody as an efficient therapy for erythroleukemia through target up regulation and antibody. *MAbs,* 2019, vol. 11 (3), 359-605
- **STOCKI, P ; SZARY, J ; RASMUSSEN, CLM ; DE-MYDCHUK, M ; NORTHALL, L ; LOGAN, DB ; GAUHAR, A ; THEI, L ; MOOS, T ; WALSH, FS et al.** Blood-brain barrier transport using a high affinity, brain-selective VNAR antibody targeting transferrin receptor 1. *The FASEB Journal.,* 2021, vol. 35 (2), e21172
- **NILSSON, B ; MOKS, T ; JANSSON, B ; AB-RAHMSÉN, L ; ELMBLAD, A ; HOLMGREN, E ; HENRICHSON, C ; JONES, TA ; UHLÉN, M.** A synthetic IgG-binding domain based on staphylococcal protein A. *Protein Engineering, Design and Selection.,* 1987, vol. 1 (2), 107-113
- **GE, YJ ; OU, YN ; DENG, YT ; WU, BS ; YANG, L ; ZHANG, YR ; CHEN, SD ; HUANG, YY ; DONG, Q ; TAN, L et al.** Prioritization of Drug Targets for Neurodegenerative Diseases by Integrating Genetic and Proteomic Data From Brain and Blood. *Biol Psychiatry.,* November 2022
- Brain Cancer Drug Discovery: Clinical Trials, Drug Classes, Targets, and Combinatorial Therapies. **SOKOLOV, A V. ; DOSTDAR, SA ; ATTWOOD, MM ; KRASILNIKOVA, AA ; ILINA, AA ; NABIEVA, ASH ; LISITSYNA, AA ; CHUBAREV, VN ; TARAS-OV, V V. ; SCHIÖTH, HB.** Pharmacol Rev. 2021, vol. 73, 1172-1203

- **KONTERMANN, RE.** Strategies to Extend Plasma Half-Lives of Recombinant Antibodies. *BioDrugs.,* 2009, vol. 23 (2), 93-109
- **YU, F ; GUDMUNDSDOTTER, L ; AKAL, A ; GUN-NERIUSSON, E ; FREJD, F ; NYGREN, PA.** An affibody-adalimumab hybrid blocks combined IL-6 and tnf-triggered serum amyloid a secretion in vivo. *MAbs,* 2014, vol. 6 (6), 1598-1607
- **JONSSON, A ; DOGAN, J ; HERNE, N ; AB-RAHMSÉN, L ; NYGREN, PÅ.** Engineering of a femtomolar affinity binding protein to human serum albumin. *Protein Engineering, Design and Selection,* 2008, vol. 21 (8), 515-527
- **MAURER, J ; JOSE, J ; MEYER, TF ; MOLEKU-LARE BIOLOGIE, A.** Autodisplay: One-Component System for Efficient Surface Display and Release of Soluble Recombinant Proteins from Escherichia coli. *JBacteriol,* 1997, vol. 179 (3), 794-804
- **ORLOVA, A ; MAGNUSSON, M ; ERIKSSON, TLJ ; NILSSON, M ; LARSSON, B ; HÖIDEN-GUTHEN-BERG, I ; WIDSTRÖM, C ; CARLSSON, J ; TOL-MACHEV, V ; STAHL, S et al.** Tumor imaging using a picomolar affinity HER2 binding Affibody molecule. *Cancer Res.,* 2006, vol. 66 (8), 4339-4348
- **HJELM, LC ; HEDHAMMAR, M ; LÖFBLOM, J.** In vitro Blood-Brain Barrier Model based on Recombinant Spider SilkProtein Nanomembranes for Evaluation of Transcytosis capability of biomolecules. *Unpublished*
- **EKLUND, M ; AXELSSON, L ; UHLÉN, M ; NY-GREN, PÅ.** Anti-idiotypic protein domains selected from protein A-based affibody libraries. *Proteins: Structure, Function and Genetics.,* 2002, vol. 48 (3), 454-462
- **MAYLE, KM ; LE, AM ; KAMEI, DT.** The intracellular trafficking pathway of transferrin. *Biochimica et Biophysica Acta (BBA) - General Subjects.,* 2012, vol. 1820 (3), 264-281
- **LAMB, JE ; RAY, F ; WARD, JH ; KUSHNER, JP ; KAPLAN, J.** Internalization and subcellular localization of transferrin and transferrin receptors in HeLa cells. *J Biol Chem.,* 1983, vol. 258 (14), 8751-8758, http://www.ncbi.nlm.nih.gov/pubmed/6305999